# EUROPEAN PATENT SPECIFICATION

(11) **EP 1 146 900 B1**
(45) Date of publication and mention of the grant of the patent: **09.08.2006**
(21) Application number: 00908388.2
(22) Date of filing: 27.01.2000
(51) Int. Cl.: A61K 39/12, A61K 39/385, A61K 48/00, A61P 31/14, C12N 15/40

(54) **DNA VACCINES AGAINST HANTAVIRUS INFECTIONS**
DNA IMPFSTOFFE GEGEN HANTAVIRUS INFEKTIONEN
VACCINS ADN DIRIGES CONTRE LES INFECTIONS A HANTAVIRUS

(30) Priority: 29.01.1999 US 117680 P
(43) Date of publication of application: 24.10.2001
(73) Proprietor: U.S. Medical Research Institute of Infectious Diseases, Frederick, MD 21702 (US)
(72) Inventor: SCHMALJOHN, Connie, S., Frederick, MD 21702 (US); HOOPER, J., W., New Market, MD 21774 (US)
(74) Representative: Ablewhite, Alan James
(86) International application number: PCT/US2000/001999
(87) International publication number: WO 2000/044406

(56) References cited:
- DONNELLY J J ET AL: "DNA vaccines." ANNUAL REVIEW OF IMMUNOLOGY, vol. 15, 1997, pages 617-648, XP000938793
- SCHMALJOHN C ET AL: "Hantaviruses: A global disease problem." EMERGING INFECTIOUS DISEASES, vol. 3, no. 2, 1997, pages 95-104, XP000938788 cited in the application
- SCHMALJOHN C S ET AL: "Antigenic subunits of Hantaan virus expressed by Baculovirus and vaccinia virus recombinants" JOURNAL OF VIROLOGY, vol. 64, no. 7, 1990, pages 3162-3170, XP000938804 cited in the application
- HOOPER J W ET AL: "DNA vaccination with hantavirus M segment elicits neutralizing antibodies and protects against Seoul virus infection." VIROLOGY, vol. 255, no. 2, 15 March 1999 (1999-03-15), pages 269-278, XP002145874
- BHARADWAJ M ET AL: "Intramuscular inoculation of Sin Nombre hantavirus cDNAs induces cellular and humoral immune responses in BALB/c mice" VACCINE, vol. 17, no. 22, 16 July 1999 (1999-07-16), pages 2836-2843, XP004171716
- DATABASE CHEMABS [Online] CHEMICAL ABSTRACTS SERVICE; ZHANG T ET AL: "Experimental study on specific cytotoxic T lymphocyte induced by nucleocapsid protein of Hantaan virus" retrieved from STN Database accession no. 133:103511 XP002145875 & ZHONGHUA WEISHENGWUXUE HE MIANYIXUE ZAZHI, vol. 20, no. 1, 2000, pages 23-25,

## Description

### INTRODUCTION

Currently, there are four known hantaviruses associated with hemorrhagic fever with renal syndrome (HFRS): Hantaan virus (HTNV), Dobrava-Belgrade virus (DOBV), Puumala virus (PUUV), and Seoul virus (SEOV). Because distinct hantaviruses are usually carried by only one principal rodent host species, their distribution is generally limited to the range of that host (reviewed in Schmaljohn and Hjelle, 1997, *Emerg*. *Infect. Dis.* 3, 95-104). HTNV, carried by *Apodemus agrarius*, is found in Asia; DOBV, carried by *Apodemus flavicollis,* and PUUV, carried by *Clethrionomys glareolus,* are found in Europe. SEOV is more widely disseminated than any other recognized hantavirus because its host, the common urban rat (*Rattus norvegicus*), is found throughout the world.

Viruses in the *Hantavirus* genus (family *Bunyaviridae*) are enveloped and contain a genome comprised of three single-stranded RNA segments designated large (L), medium (M), and small (S) based on size (reviewed in Schmaljohn, 1996, In The Bunyaviridae Ed. R. M. Elliott. New York, Plenum Press p. 63-90). The hantavirus L segment encodes the RNA dependent RNA polymerase, M encodes two envelope glycoproteins (G1 and G2), and S encodes the nucleocapsid protein (N).

A number of inactivated HFRS vaccines derived from cell culture or rodent brain were developed and tested in Asia (Lee *et al*., 1990, *Arch. Virol*., Suppl. 1, 35-47; Song *et al.,* 1992, *Vaccine* 10, 214-216; Lu *et al.,* 1996, *J. Med. Virol.* 49, 333-335). Other vaccine approaches involving recombinant DNA technology include: vaccinia-vectored vaccines (Schmaljohn *et al*. 1990, *J. Virol*. 64, 3162-3170; Schmaljohn *et al.* 1992, *Vaccine* 10, 10-13; Xu *et al*. 1992, *Am. J. Trop. Med. Hyg*. 47, 397-404), protein subunit vaccines expressed in bacteria or insect cells (Schmaljohn *et al*. 1990, supra; Yoshimatsu *et al.,* 1993, *Arch. Virol*. 130, 365-376; Lundkvist *et al.*, 1996, *Virology* 216, 397-406), and a hepatitis core antigen-based recombinant vaccine (Ulrich *et al*., 1998, *Vaccine* 16, 272-280).

Hantavirus G1, G2, and N proteins are known to elicit immune responses; however, the relative importance these proteins play in providing protection remains an area of investigation. Vaccination with vaccinia recombinants expressing the M segment of either HTNV or SEOV elicited neutralizing antibodies and protected rodents against infection with both HTNV and SEOV, suggesting that an immune response to G1-G2 alone can confer protection (Schmaljohn *et al*. 1990, supra; Xu *et al.* 1992, supra; Chu *et al*. 1995, *J. Virol*. 69, 6417-6423). Similarly, vaccination with G1-G2 protein expressed in insect cells (baculovirus recombinant virus system) elicited neutralizing antibodies and protected hamsters from infection with HTNV (Schmaljohn *et al*. 1990, supra). In both the vaccinia and baculovirus systems, vaccination with G1-G2 provided more complete protection than G1 or G2 alone (Schmaljohn *et al*. 1990, supra). Neutralizing antibody responses to G1-G2 in the aforementioned vaccine studies correlated with protection, suggesting that neutralizing antibodies play an important role in preventing hantavirus infection. Passive transfer of neutralizing monoclonal antibodies (MAbs) specific to either G1 or G2 protected hamsters against HTNV infection (Schmaljohn *et al*., 1990, supra; Arikawa *et al.*, 1992, *J. Gen. Virol*. 70, 615-624), supporting the idea that neutralizing antibodies alone can confer protection. The N protein also plays a role in protecting against hantavirus infection. Vaccination with N expressed in bacteria, insect cells, or as chimeric hepatitis B virus (HBV) core particles protected rodents from hantavirus infection (Schmaljohn *et al.,* 1990, supra; Yoshimatsu *et al*. 1993, supra; Lundkvist *et al*., 1996, supra; Ulrich *et al*., 1998, supra). Vaccination with vaccinia recombinants expressing the S segment were less conclusive. A construct expressing the HTNV S segment did not protect hamsters from HTNV infection, possibly due to low N expression levels (Schmaljohn *et al*. 1990, supra); and a construct expressing the S segment of SEOV protected three of four gerbils from SEOV infection (Xu *et al*. 1992, supra).

Recombinant vaccinia virus vaccines and vaccinia-based vaccines present disadvantages including the potential for disseminated infection, especially in immunocompromised individuals, since the vaccines consist of live virus. Also, vaccination with these viruses can result in a lesion (pock) that contains infectious virus. Virus from these lesions can be inadvertently spread to other sites (e.g., eyes) or to other individuals. Other drawbacks of vaccinia-based vaccines include discomfort due to swollen lymphnodes and scarring at the site of inoculation.

DNA vaccination mimicks the de novo antigen production and MHC class I-restricted antigen presentation obtainable with live vaccines, without the risks of pathogenic infection, as discussed in J.H. Donnelly et al., 1997, *Annu.Rev.Immunol*. 15, 617-648.

This vaccine approach is advantageous over subunit vaccine which do not elicit a cytotoxic response necessary to prevent the establishment of infection or disease. Also, this DNA vaccine approach allows delivery to mucosal tissues which may aid in conferring resistance to viral introduction since entry of the virus may be through mucosal tissues.

### SUMMARY OF THE INVENTION

In this report, we describe a new recombinant DNA vaccine approach that involves vaccination with naked DNA expressing individual hantavirus genome segment cDNAs. Naked DNA vaccination involves delivery of plasmid DNA constructs with a gene(s) of interest into the tissue of the vaccinee (reviewed in Robinson and Torres, 1997, *Semin. Immunol*. 9, 271-283; and Gregoriadis, 1998, *Pharm. Res.* 15, 661-670). The gene(s) of interest, in our case, a hantavirus genome segment, is controlled by a mammalian or virus promoter (e.g., the cytomegalovirus immediate early promoter) that facilitates expression of the naked DNA gene product(s) within the vaccinee's cells. This intracellular expression can elicit both humoral and cell-mediated immune responses (Robinson and Torres, 1997, supra; and Gregoriadis, 1998, supra). Methods of DNA delivery include needle inoculation, oral or pulmonary delivery, and inoculation by particle bombardment (i.e., gene gun). DNA vaccination by each of these methods elicits protective immunity against many different pathogens including numerous viruses (Robinson and Torres, 1997,supra; and Gregoriadis, 1998, supra). However, neither an immune response against hantaviruses nor protection against hantavirus infection have been demonstrated using a DNA vaccine.

In this report, we demonstrate that naked DNA vaccination with the SEOV M or S genome segment elicits SEOV-specific antibody responses in rodents. More importantly, we demonstrate that DNA vaccination with the SEOV M segment elicits neutralizing antibodies and protects hamsters against SEOV infection.

Therefore, it is one object of the present invention to provide a hantavirus DNA vaccine comprising a hantavirus genome segment. More specifically, the present invention relates to an SEOV hantavirus DNA vaccine comprising the SEOV M genome segment specified in SEQ ID NO:1 or comprising the SEOV S segment specified in SEQ ID NO:2.

It is another object of the present invention to provide a method for eliciting in a subject an immune response against hantavirus, the method comprising administering to a subject a DNA fragment comprising a genome segment of hantavirus. More specifically, the present invention relates to a method for eliciting an immune response against SEOV hantavirus by providing the M or S genome segment of SEOV. Cross protection against other hantaviruses such as Hantaan virus and Dobrava are also an object of this invention.

In one aspect of the invention, the DNA vaccine is delivered by coating a small carrier particle with the DNA vaccine and delivering the DNA-coated particle into an animal's epidermal tissue via particle bombardment. This method may be adapted for delivery to either epidermal or mucosal tissue, or delivery into peripheral blood cells, and thus may be used to induce humoral, cell-mediated, and secretory immune reponses in the vaccinated individual.

The DNA vaccine according to the present invention is inherently safe, is not painful to administer, and should not result in adverse side effects to the vaccinated individual. In addition, the invention does not require growth or use of hantavirus, which may be spread by aerosol transmission.

### BRIEF DESCRIPTION OF THE DRAWINGS

These and other features, aspects, and advantages of the present invention will become better understood with reference to the following description, appended claims, and accompanying drawings where:

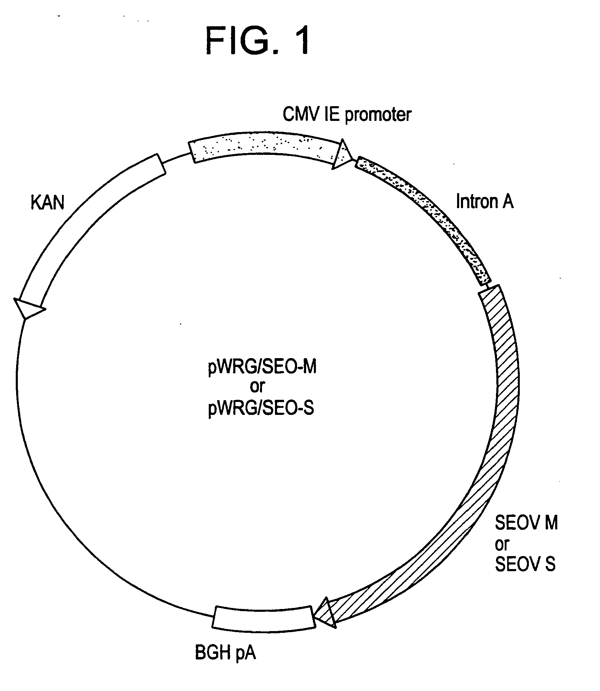

**FIG. 1.** SEOV naked DNA expression constructs. The SEOV M or S genome segment was amplified by RT-PCR and cloned into *Not*I and *Bam*HI sites of pWRG7077 (PowderJect Vaccines, Inc., Madison, Wisconsin, described in Schmaljohn et al., 1997, supra). Characteristics of pWRG7077 are similar to those of pWRG1602 described previously (Dimmock, N. J., 1995, *Med. Virol.* 5: 165) and include a human cytomegalovirus early promoter (CMV IE promoter) and intron A, a bovine growth hormone transcription terminator and polyadenylation signal (BGH pA), and a kanamycin resistance gene. CMV IE promoter: cytomegalovirus immediate early promoter and intron A, BGH pA: Bovine growth hormone polyadenylation signal, KAN: kanamycin antibiotic resistance gene.

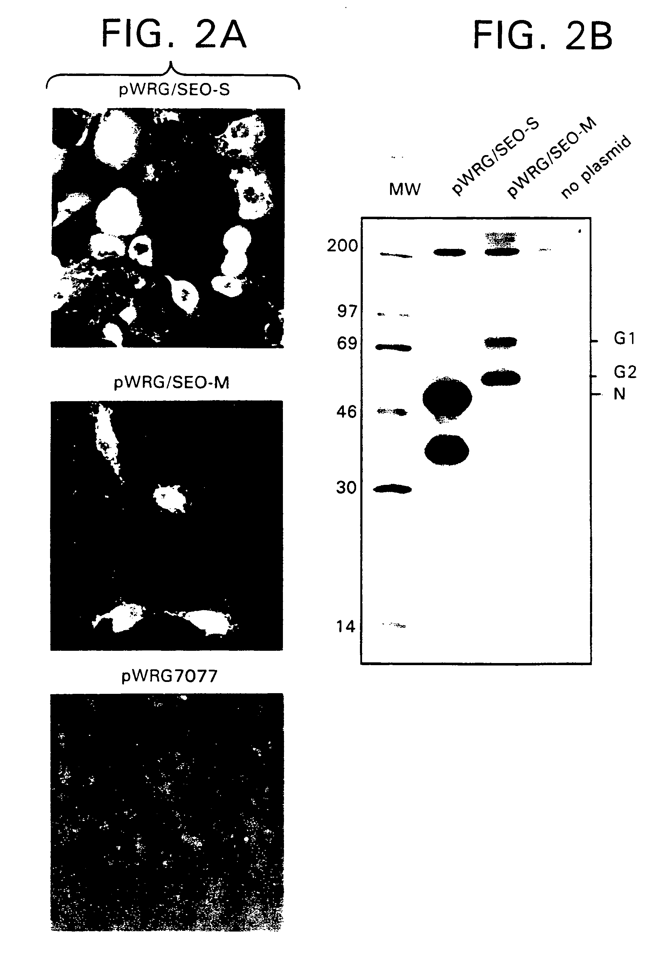

**FIG. 2 A and B**. Transient expression from pWRG/SEO-M (SEQ ID NO:3) and pWRG/SEO-S (SEQ ID NO:4). A) IFAT were performed on COS cell monolayers transfected with the SEOV M or S constructs. pWRG/7077-transfected monolayers served as negative controls. Anti-SEOV polyclonal rabbit antisera was used as the primary antibody. B) COS cells were transfected with the indicated plasmid and radiolabeled expression products were immunoprecipitated with anti-SEOV polyclonal rabbit serum. Molecular size markers (MW) in kDa are shown on the left, and the position of the SEOV G1, G2, and N proteins are shown on the right.

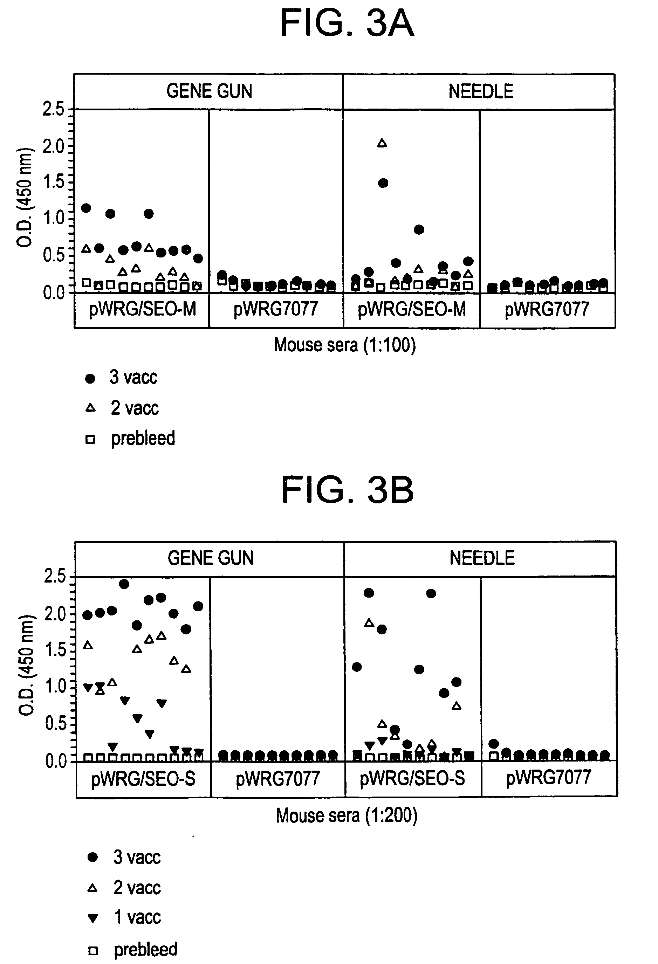

**FIG. 3A and 3B.** Antibody responses (ELISA) of mice vaccinated with SEOV naked DNA by gene gun or intramuscular needle inoculation. The antibody responses to vaccination with pWRG/SEO-M (panel A) or pWRG/SEO-S (panel B) by gene gun or intramuscular needle inoculation were evaluated by ELISA. Mice vaccinated with negative control plasmid pWRG7077 were evaluated in both ELISAs. Prebleed sera and sera collected 4 weeks after the first vaccination (1 vacc), 4 weeks after the second vaccination (2 vacc), 3 weeks after the final vaccination (3 vacc) were all tested in a single assay. Symbols represent the average values of individual mice serum samples assayed in duplicate.

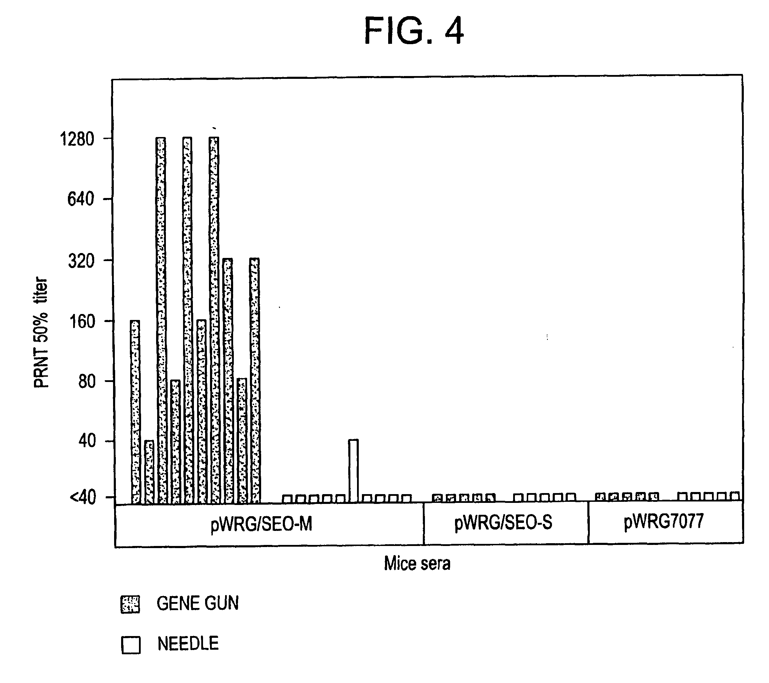

**FIG. 4.** Neutralizing antibody responses of mice vaccinated with SEOV naked DNA. PRNT were performed on mice sera collected 3 weeks after the third vaccination by either gene gun or intramuscular needle injection (needle). Bars represent sera from individual mice vaccinated with the indicated immunogen. Sera were heat inactivated (56°C, 30 min) and the assay was performed in the presence of 5% guinea pig complement. PRNT50% titers are expressed as reciprocal of the highest serum dilution resulting in a 50% reduction in plaque number.

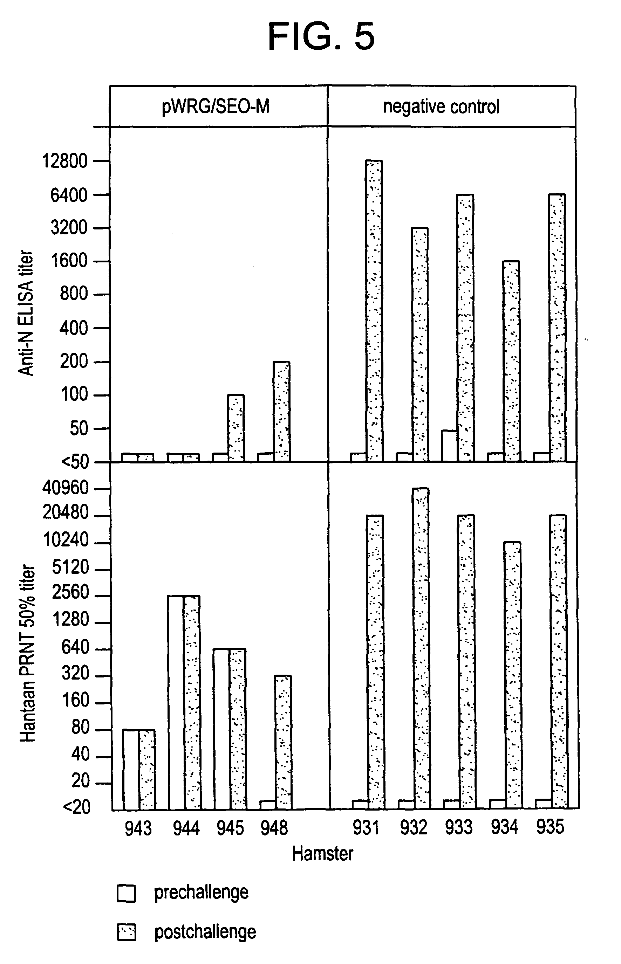

**FIG. 5.** Vaccination with pWRG/SEO-M cross-protects against infection with Hantaan virus. Groups of 4-5 hamsters were vaccinated with either pWRG/SEO-M or negative control plasmid (pWRG7077) as described in Methods below. Three weeks after the final vaccination, prechallenge serum samples were obtained and the hamsters were challenged with 1,000 PFU of Hantaan virus. Twenty-eight days after challenge postchallenge serum samles were obtained. Pre and postchallenge serum samples were evaluated by anti-N ELISA to detect antibody to nucleocapsid, and by a Hantaan plaque reduction neutralization test (PRNT) to detect Hantaan neutralizing antibodies. ELISA titers represent the lowest reciprocal dilution that resulted in an O.D. value that was greater than the background O.D. plus three standard deviations. PRNT50% titers represent the lowest reciprocal dilution that resulted in a 50% reduction in plaque number in the absence of serum.

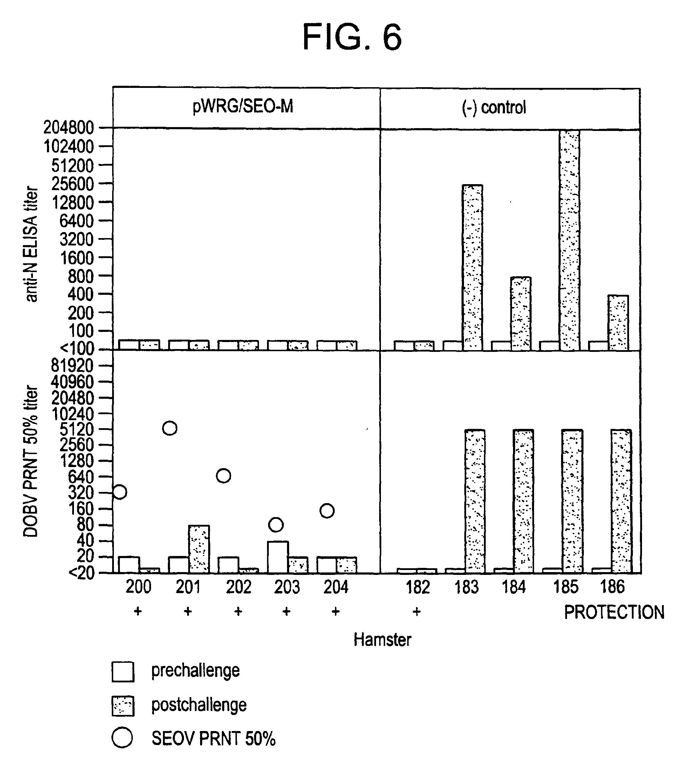

**FIG. 6**. Vaccination with pWRG/SEO-M cross-protects against infection with Dobrava virus. Groups of 5 hamsters were vaccinated with either pWRG/SEO-M or negative control plasmid (pWRG7077) as described in Methods. Three weeksafter the final vaccination, prechallenge serum samples were obtained and the hamsters were challenged with 1,000 PFU of Dobrava virus. Twenty-eight days after challenge postchallenge serum samples were obtained. Pre and postchallenge serum samples were evaluated by anti-N ELISA to detect antibody to nucleocapsid, and by a Dobrava plaque reduction neutralization test (PRNT) to detect Dobrava neutralizing antibodies. ELISA titers represent the lowest reciprocal dilution that resulted in an O.D. value that was greater than the background O.D. plus three standard deviations. PRNT50% titers represent the lowest reciprocal dilution that resulted in a 50% reduction in plaque number in the absence of serum. The Seoul neutralizing prechallenge antibody titers (elicted by the vaccine) were also measured. The Seoul virus PRNT80% values are shown as empty circles.

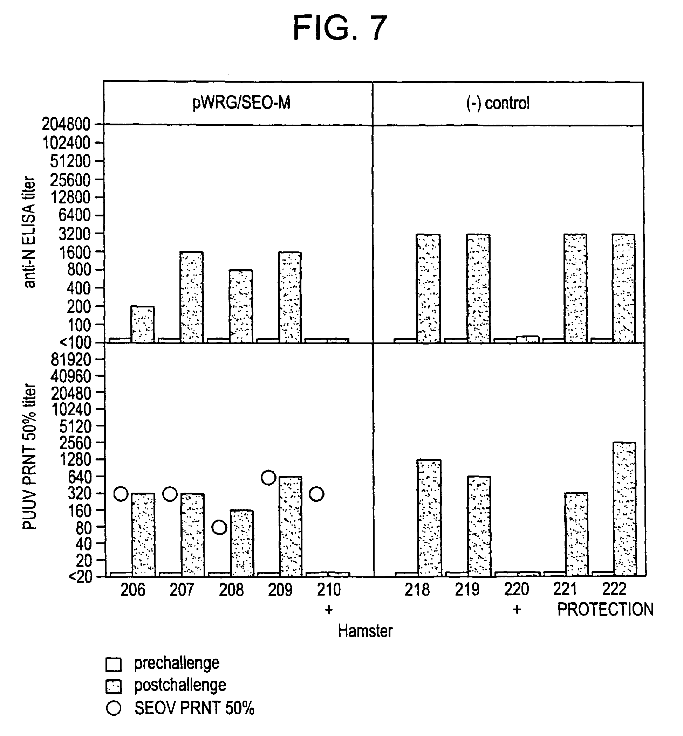

**FIG. 7**. Vaccination with pWRG/SEO-M fails to cross-protect against infection with Puumala virus. Groups of 5 hamsters were vaccinated with either pWRG/SEO-M or negative control plasmid (pWRG7077) as described in Methods. Three weeks after the final vaccination, prechallenge serum samples were obtained and the hamsters were challenged with 1,000 PFU of Puumala virus. Twenty-eight days after challenge postchallenge serum samples were obtained. Pre and postchallenge serum samples were evaluated by anti-N ELISA to detect antibody to nucleocapsid, and by a Puumala plaque reduction neutralization test (PRNT) to detect Puumala neutralizing antibodies . ELISA titers represent the lowest reciprocal dilution that resulted in an O.D. value that was greater than the background O.D. plus three standard deviations. PRNT50% titers represent the lowest reciprocal dilution that resulted in a 50% reduction in plaque number in the absence of serum. The Seoul neutralizing prechallenge antibody titers (elicted by the vaccine) were also measured. The Seoul virus PRNT80% values are shown as empty circles.

### DETAILED DESCRIPTION

In this application is described a composition and method for the vaccination of individuals against hantavirus. The method comprises delivery of a DNA encoding a hantavirus antigen to cells of an individual such that the antigen is expressed in the cell and an immune response is induced in the individual.

DNA vaccination involves administering antigen-encoding polynucleotides *in vivo* to induce the production of a correctly folded antigen(s) within the target cells. The introduction of the DNA vaccine will cause to be expressed within those cells the structural protein determinants associated with the pathogen protein or proteins. The processed structural proteins will be displayed on the cellular surface of the transfected cells in conjunction with the Major Histocompatibility Complex (HMC) antigens of the normal cell. Even when cell-mediated immunity is not the primary means of preventing infection, it is likely important for resolving established infections. Furthermore, the structural proteins released by the expressing transfected cells can also be picked up by antigen-presenting cells to trigger systemic humoral antibody responses.

In order to achieve the immune response sought, a DNA vaccine construct capable of causing transfected cells of the vaccinated individual to express one or more major viral antigenic determinant is necessary. This can be done by identifying regions of the viral genome which code for viral glycoproteins or capsid components, and joining such coding sequences to promoters capable of expressing the sequences in cells of the vaccinee. Alternatively, the viral genome itself, or parts of the genome, can be used.

In one embodiment, the present invention relates to a DNA or cDNA segment which encodes an antigen from a hantavirus. By hantavirus is meant any of the Hemmorhagic fever with renal syndrome (HFRS) hantavirus such as Hantaan virus (HTNV), Dobrava-Belgrade virus (DOBV), Puumala virus (PUUV), and Seoul virus (SEOV), as well as hantavirus pulmonary syndrome (HPS) hantaviruses such as Sin Nombre virus (SNV), Black Creek Canal virus (BCCV), Bayou virus (BAYV), New York virus (NYV), Andes virus (ANDV), Laguna Negra virus (LNV), and any other hantavirus known to cause disease in humans.

More specifically, a hantavirus genome M segment encoding two envelope glycoproteins (G1 and G2) (SEQ ID NO:1), and a hantavirus genome S segment encoding nucleocapsid protein (SEQ ID NO:2) were deduced from the SEOV, strain SR-11 (Kitamura, T. et al. 1983, *Jpn. J. Med. Sci. Biol.* 36, 17-25) viral genome (Arikawa, J. et al. 1990, *Virology* 176, 114-125). The M segment (specified in SEQ ID NO:1), deposited in GenBank accession no. M34882, corresponds to Seoul (SR-11) M gene from nucleotide 2 to 3602. The S segment (SEQ ID NO:2), having Genbank accession no. M34881, corresponds to Seoul (SR-11) S gene from nucleotide 2 to 1699.

DNA or polynucleotide sequences to which the invention also relates include fragments of the M or S gene segment from other Hantaviruses containing protective epitopes or antigenic determinants. Such epitopes, which may be conformational, may include sequences from G1 and/or G2 since monoclonal antibodies to both G1 and G2 have been shown to neutralize virus and protect rodents in passive protection experiments. Additionally, the amino terminus of N is highly immunogenic and others have shown that other methods of vaccination with the amino terminal region can confer protection (Lundvist, 1996, supra; Ulrich, 1998, supra).

The derived sequence is not necessarily physically derived from the nucleotide sequence shown in SEQ ID NO:1 or SEQ ID NO:2, but may be generated in any manner, including for example, chemical synthesis or DNA replication or reverse transcription or transcription, which are based on the information provided by the sequence bases in the region(s) from which the polynucleotide is derived. In addition, combinations of regions corresponding to that of the designated sequence may be modified in ways known in the art to be consistent with an intended use. The sequences of the present invention can be used in diagnostic assays such as hybridization assays and polymerase chain reaction (PCR) assays for the detection of Hantavirus.

RT-PCR cloning of the M and S genome segments of SEOV, strain SR-11, was described previously (Arikawa et al., 1990, Virology 176, 114-125). A DNA fragment containing Seoul (SR-11) M gene from nucleotide 2 to 3602 was excised from YMIS/SR 11-M (Arikawa, 1990, supra) using Bgl II and Bam HI. This fragment was ligated into the Bam HI site of pWRG7077 resulting in pWRG/SEO-M (SEQ ID NO:3). pWRG/SEO-M contains additional nucleotides derived from cloning vector baculovirus transfer vector YMIS.

A DNA fragment containing Seoul (SR-11) S gene from nucleotide 2 to 1699 was excised from pBSKS+/SR11-S (Arikawa, 1990, supra) using EcoRI. This fragment was subjected to Klenow to blunt the restriction sites and then ligated into Klenow-blunted Not I site of pWRG7077 (provided by Powderject, Inc., Madison, Wisconsin) resulting in pWRG/SEO-S (SEQ ID NO:4). pWRG/SEO-S contains additional nucleotides derived from cloning vector pBSKS+.

It is understood in the art that certain changes to the nucleotide sequence employed in a genetic construct have little or no bearing on the proteins encoded by the construct, for example due to the degeneracy of the genetic code. Such changes result either from silent point mutations or point mutations that encode different amino acids that do not appreciably alter the behavior of the encoded protein. It is also understood that portions of the coding region can be eliminated without affecting the ability of the construct to achieve the desired effect, namely induction of a protective immune response against hantavirus. It is further understood in the art that certain advantageous steps can be taken to increase the antigenicity of an encoded protein by modifying its amino acid composition. Such changes in amino acid composition can be introduced by modifying the genetic sequence encoding the protein. It is contemplated that all such modifications and variations of the M and S segments of hantavirus are equivalents within the scope of the present invention.

The DNA encoding the desired antigen can be introduced into the cell in any suitable form including, a linearized plasmid, a circular plasmid, a plasmid capable of replication, an episome, RNA, etc. Preferably, the gene is contained in a plasmid. In a particularly preferred embodiment, the plasmid is an expression vector. Individual expression vectors capable of expressing the genetic material can be produced using standard recombinant techniques. Please see e.g., Maniatis *et al*., 1985 Molecular Cloning: A Laboratory Manual or DNA Cloning, Vol. I and II (D. N. Glover, ed., 1985) for general cloning methods.

Therefore, in another embodiment, the present invention relates to a recombinant DNA molecule that includes a vector and a DNA sequence as described above. The vector can take the form of a plasmid such as pCRII (Invitrogen) or pJW4303 (Konishi, E. *et al*., 1992, *Virology* 188:714), or any expression vector such as viral vectors e.g. adenovirus or Venezuelan equine encephalitis virus and others known in the art. Preferably, a promoter sequence operable in the target cell is operably linked to the DNA sequence. Several such promoters are known for mammalian systems which may be joined 5', or upstream, of the coding sequence for the encoded protein to be expressed. A suitable promoter is the human cytomegalovirus immediate early promoter. A downstream transcriptional terminator, or polyadenylation sequence, such as the polyA addition sequence of the bovine growth hormone gene, may also be added 3' to the protein coding sequence.

A suitable construct for use in the method of the present invention is pWRG7077 (4326 bp)(PowderJect Vaccines, Inc., Madison, WI), Figure 1. pWRG7077 includes a human cytomegalovirus (hCMV) immediate early promoter (IE) and a bovine growth hormone polyA addition site. Between the promoter and the polyA addition site is Intron A, a sequence that naturally occurs in conjunction with the hCMV IE promoter that has been demonstrated to increase transcription when present on an expression plasmid. Downstream from Intron A, and between Intron A and the polyA addition sequence, are unique cloning sites into which the hantavirus M or S DNA can be cloned. Also provided on pWRG7077 is a gene that confers bacterial host-cell resistance to kanamycin. Any of the fragments that encode hantavirus G1 or G2 or nucleocapsid peptides can be cloned into one of the cloning sites in pWRG7077, using methods known to the art.

In a further embodiment, the present invention relates to host cells stably transformed or transfected with the above-described recombinant DNA construct. The host cell can be prokaryotic such as *Bacillus* or *E. coli,* or eukaryotic such a *Saccharomyces* or *Pichia,* or mammalian cells or insect cells. The vector containing the hantavirus sequence is expressed in the bacteria and the expressed product used for diagnostic procedures or as a vaccine. Please see e.g., Maniatis *et al.,* 1985 Molecular Cloning: A Laboratory Manual or DNA Cloning, Vol. I and II (D. N. Glover, ed., 1985) for general cloning methods. The DNA sequence can be present in the vector operably linked to a highly purified IgG molecule, an adjuvant, a carrier, or an agent for aid in purification of hantavirus proteins or peptides. The transformed or transfected host cells can be used as a source of DNA sequences described above. When the recombinant molecule takes the form of an expression system, the transformed or transfected cells can be used as a source of the protein or peptide encoded by the DNA. The DNA can be used as circular or linear, or linearized plasmid as long as the hantavirus sequences are operably linked to a promoter which can be expressed in the transfected cell.

In this application we describe the elicitation of protective immunity to hantaviruses by DNA vaccines. The DNA can be delivered by injection into the tissue of the recipient, oral or pulmonary delivery and inoculation by particle bombardment (i.e., gene gun). Any of these methods can be used to deliver DNA as long as the DNA is expressed and the desired antigen is made in the cell. Two methods are exemplified in this application, both shown to be successful in eliciting a protective immune response in the vaccinee.

To deliver DNA vaccines by particle bombardment, we chose to use the *PowderJect-XR™* gene gun device described in WO 95/19799, 27 July 1995. Other instruments are available and known to people in the art. This instrument, which delivers DNA-coated gold beads directly into epidermal cells by high-velocity particle bombardment, was shown to more efficiently induce both humoral and cell-mediated immune responses, with smaller quantities of DNA, than inoculation of the same DNAs by other parenteral routes (Eisenbraun, M. *et al.,* 1993, *DNA Cell. Biol.* 12: 791; Fynan, E. F. *et al*., 1993, *Proc. Natl. Acad. Sci. USA* 90: 11478; Haynes, J. R. *et al.,* 1994, *AIDS Res. Hum. Retroviruses* 10: Suppl. 2:S43; Pertmer, T. M. *et al*., 1995, *Vaccine* 13: 1427). Epidermal inoculation of the DNA candidate vaccines also offers the advantages of gene expression in an immunologically active tissue that is generally exfoliated within 15 to 30 days, and which is an important natural focus of viral replication after tick-bite (Bos, J. D., 1997, *Clin. Exp. Immunol*. 107 Suppl. 1:3; Labuda, M. *et al*., 1996, *Virology* 219:357; Rambukkana, A. *et al.,* 1995, *Lab. Invest.* 73:521; Stingl, G., 1993, *Recent Results Cancer Res*. 128:45). Candidate vaccines include particles having M genome segments, including G1 and/or G2, from different HFRS viruses such as Seoul virus, Hantaan virus, Pumuula virus, and Dobrava virus, as well as Hantavirus pulmonary syndrome virus such as Sin Nombre virus, Black Creek Canal virus, Bayou virus, New York virus, Andes virus, and Laguna Negra virus. The DNA segments from different viruses can be on different particles or on the same particle, whichever results in the desired immune response. In addition, the present invention relates to a vaccine comprising one or more DNAs from different hantaviruses. Such a vaccine is referred to as a multivalent vaccine. The vaccine is designed to protect against pathologies resulting from exposure to one or several hantaviruses. The vaccine can also be combined with reagents which increase the antigenicity of the vaccine, or reduce its side effects.

The technique of accelerated particles gene delivery or particle bombardment is based on the coating of DNA to be delivered into cells onto extremely small carrier particles, which are designed to be small in relation to the cells sought to be transformed by the process. The DNA sequence containing the desired gene can be simply dried onto a small inert particle. The particle may be made of any inert material such as an inert metal (gold, silver, platinum, tungsten, etc.) or inert plastic (polystyrene, polypropylene, polycarbonate, etc.). Preferably, the particle is made of gold, platinum or tungsten. Most preferably, the particle is made of gold. Suitably, the particle is spherical and has a diameter of 0.5 to 5 microns, preferably 1 to 3 microns.

The DNA sequence containing the desired gene prepared in the form suitable for gene introduction can be simply dried onto naked gold or tungsten pellets. However, DNA molecules in such a form may have a relatively short period of stability and may tend to degrade rather rapidly due to chemical reactions with the metallic or oxide substrate of the particle itself. Thus, if the carrier particles are first coated with an encapsulating agent, the DNA strands have greatly improved stability and do not degrade significantly even over a time period of several weeks. A suitable encapsulating agent is polylysine (molecular weight 200,000) which can be applied to the carrier particles before the DNA molecules are applied. Other encapsulating agents, polymeric or otherwise, may also be useful as similar encapsulating agents, including spermidine. The polylysine is applied to the particles by rinsing the gold particles in a solution of 0.02% polylysine and then air drying or heat drying the particles thus coated. Once the metallic particles coated with polylysine were properly dried, DNA strands are then loaded onto the particles.

The DNA is loaded onto the particles at a rate of between 0.5 and 30 micrograms of DNA per milligram of gold bead spheres. A preferable ratio of DNA to gold is 0.5-5.0 ug of DNA per milligram of gold. A sample procedure begins with gamma irradiated (preferably about 30 kGy) tefzel tubing. The gold is weighed out into a microfuge tube, spermidine (free base) at about 0.05 M is added and mixed, and then the DNA is added. A 10% CaCl solution is incubated along with the DNA for about 10 minutes to provide a fine calcium precipitate. The precipitate carries the DNA with it onto the beads. The tubes are microfuged and the pellet resuspended and washed in 100% ethanol and the final product resuspeded in 100% ethanol at 0.0025mg/ml PVP. The gold with the DNA is then applied onto the tubing and dried.

The general approach of accelerated particle gene transfection technology is described in U.S. Patent No. 4,945,050 to Sanford. An instrument based on an improved variant of that approach is available commercially from PowderJect Vaccines, Inc., Madison Wisconsin, and is described in WO 95/19799. All documents cited herein *supra* and infra are hereby incorporated in their entirety by reference thereto. Briefly, the DNA-coated particles are deposited onto the interior surface of plastic tubing which is cut to a suitable length to form sample cartridges. A sample cartridge is placed in the path of a compressed gas (e.g., helium at a pressure sufficient to dislodge the particles from the cartridge e.g., 350-400 psi). The particles are entrained in the gas stream and are delivered with sufficient force toward the target tissue to enter the cells of the tissue. Further details are available in the published apparatus application.

The coated carrier particles are physically accelerated toward the cells to be transformed such that the carrier particles lodge in the interior of the target cells. This technique can be used either with cells in vitro or in vivo. At some frequency, the DNA which has been previously coated onto the carrier particles is expressed in the target cells. This gene expression technique has been demonstrated to work in prokaryotes and eukaryotes, from bacteria and yeasts to higher plants and animals. Thus, the accelerated particle method provides a convenient methodology for delivering genes into the cells of a wide variety of tissue types, and offers the capability of delivering those genes to cells in situ and in vivo without any adverse impact or effect on the treated individual. Therefore, the accelerated particle method is also preferred in that it allows a DNA vaccine capable of eliciting an immune response to be directed both to a particular tissue, and to a particular cell layer in a tissue, by varying the delivery site and the force with which the particles are accelerated, respectively. This technique is thus particularly suited for delivery of genes for antigenic proteins into the epidermis.

A DNA vaccine can be delivered in a non-invasive manner to a variety of susceptible tissue types in order to achieve the desired antigenic response in the individual. Most advantageously, the genetic vaccine can be introduced into the epidermis. Such delivery, it has been found, will produce a systemic humoral immune response.

To obtain additional effectiveness from this technique, it may also be desirable that the genes be delivered to a mucosal tissue surface, in order to ensure that mucosal, humoral and cellular immune responses are produced in the vaccinated individual. There are a variety of suitable delivery sites available including any number of sites on the epidermis, peripheral blood cells, i.e. lymphocytes, which could be treated in vitro and placed back into the individual, and a variety of oral, upper respiratory, and genital mucosal surfaces.

Gene gun-based DNA immunization achieves direct, intracellular delivery of DNA, elicits higher levels of protective immunity, and requires approximately three orders of magnitude less DNA than methods employing standard inoculation.

Moreover, gene gun delivery allows for precise control over the level and form of antigen production in a given epidermal site because intracellular DNA delivery can be controlled by systematically varying the number of particles delivered and the amount of DNA per particle. This precise control over the level and form of antigen production may allow for control over the nature of the resultant immune response.

The term transfected is used herein to refer to cells which have incorporated the delivered foreign DNA vaccine, whichever delivery technique is used.

It is herein disclosed that when inducing cellular, humoral, and protective immune repsonses after DNA vaccination the preferred target cells are epidermal cells, rather than cells of deeper skin layers such as the dermis. Epidermal cells are preferred recipients of DNA vaccines because they are the most accessible cells of the body and may, therefore, be immunized non-invasively. Secondly, in addition to eliciting a humoral immune response, DNA immunized epidermal cells also elicit a cytotoxic immune response that is stronger than that generated in sub-epidermal cells. Delivery to epidermis also has the advantages of being less invasive and delivering to cells which are ultimately sloughed by the body.

Although it can be desirable to induce an immune response by delivering genetic material to a target animal, merely demonstrating an immune response is not necessarily sufficient to confer protective advantage on the animal. What is important is to achieve a protective immune response that manifests itself in a clinical difference. That is, a method is effective only if it reduces the severity of the disease symptoms. It is preferred that the immunization method be at least 20% effective in preventing death in an immunized population after challenge with hantavirus. More preferably, the vaccination method is 50% or more effective, and most preferably 70-100% effective, in preventing death in an immunized population. The vaccination method is shown herein to be 100% effective in the hamster model for hantavirus. Hamsters have been used extensively as the laboratory model of choice for assessment of protective immune responses to hantaviruses. In contrast, unimmunized animals are uniformly infected by challenge with hantavirus. Our results indicate that vaccination with SEOV M genome segment protects against Hantaan virus (HTNV), and against Dobrava virus (DBOV).

Generally, the DNA vaccine administered may be in an amount of about 1-5 ug of DNA per dose and will depend on the subject to be treated, capacity of the subject's immune system to develop the desired immune response, and the degree of protection desired. Precise amounts of the vaccine to be administered may depend on the judgement of the practitioner and may be peculiar to each subject and antigen.

The vaccine for eliciting an immune response against one or more viruses, may be given in a single dose schedule, or preferably a multiple dose schedule in which a primary course of vaccination may be with 1-10 separate doses, followed by other doses given at subsequent time intervals required to maintain and or reinforce the immune response, for example, at 1-4 months for a second dose, and if needed, a subsequent dose(s) after several months. Examples of suitable immunization schedules include: (i) 0, 1 months and 6 months, (ii) 0, 7 days and 1 month, (iii) 0 and 1 month, (iv) 0 and 6 months, or other schedules sufficient to elicit the desired immune responses expected to confer protective immunity, or reduce disease symptoms, or reduce severity of disease.

In another embodiment, the present invention provides reagents useful for carrying out the present process. Such reagents comprise a DNA fragment containing M or S or both gene segments from hantavirus, and a small, inert, dense particle. The DNA fragment, and dense particle are those described above.

Preferably, the DNA is frozen or lyophilized, and the small, inert, dense particle is in dry powder. If a coating solution is used, the dry ingredients for the coating solution may be premixed and premeasured and contained in a container such as a vial or sealed envelope.

The present invention also provides kits which are useful for carrying out the present invention. The present kits comprise a first container means containing the above-described frozen or lyophilized DNA. The kit also comprises a second container means which contains the coating solution or the premixed, premeasured dry components of the coating solution. The kit also comprises a third container means which contains the small, inert, dense particles in dry powder form or suspended in 100% ethanol. These container means can be made of glass, plastic or foil and can be a vial, bottle, pouch, tube, bag, etc. The kit may also contain written information, such as procedures for carrying out the present invention or analytical information, such as the amount of reagent (e.g. moles or mass of DNA) contained in the first container means. The written information may be on any of the first, second, and/or third container means, and/or a separate sheet included, along with the first, second, and third container means, in a fourth container means. The fourth container means may be, e.g. a box or a bag, and may contain the first, second, and third container means.

Other features of the invention will become apparent in the course of the following descriptions of exemplary embodiments which are given for illustration of the invention and are not intended to be limiting thereof.

The following materials and method were used in the examples below.

### MATERIALS AND METHODS

### Viruses, cells, and medium

SEOV, strain SR-11 (Kitamura *et al*., 1983, *Jpn. J. Med. Sci. Biol.* 36, 17-25) was propagated in Vero E6 cells (Vero C1008; ATCC CRL 1586). Transient expression experiments were performed with COS cells (COS-7; ATTC CRL1651). Both cell types were maintained in Eagle's minimal essential medium with Earle's salts (EMEM) containing 10% fetal bovine serum, 10 mM HEPEs pH 7.4; and antibiotics (penicillin [100 U/ml], streptomycin [100 µg/ml], and gentamycin [50 µg/ml])(cEMEM).

### Construction of SEOV M and S naked DNA plasmids

RT-PCR cloning of the M and S genome segments of SEOV, strain SR-11, was described previously (Arikawa *et al.,* 1990, *Virology* 176, 114-125). The cDNA representing each genome segment was subcloned into the *Not* I or *Bam* HI site of a previously described naked DNA vector pWRG7077 (Schmaljohn *et al*., 1997, *J. Virol*. 64, 3162-3170). Plasmid DNA was purified using Qiagen maxiprep DNA purification kits according to the manufacturer's directions (Cat. no. 12163, Qiagen). The DNA we used for both intramuscular needle inoculation and epidermal gene gun inoculation was not prepared using endo-free qiagen DNA purification kits, and therefore, the DNA was not endotoxin-free (Qiagen plasmid kits yield -9.3 endotoxin units per µg DNA [QIAGEN Plasmid Purification Handbook 01.97]). To control for possible immunostimulatory effects of endotoxin, our negative control plasmid DNA was prepared in precisely the same was as the vaccine candidates.

### Indirect fluorescent antibody test (IFAT)

IFAT was a modification of a previously described procedure (Kamrud *et al.,* 1995, *Exp. Parasitol*. 81, 394-403). COS cells grown on 15mm glass cover slips in 12-well cell culture plates were transfected with 1 µg of plasmid DNA using Fugene6 (Boehringer Mannheim) as described by the manufacturer. Twenty-four hr posttransfection, cover slips were rinsed once with PBS (pH 7.4), fixed with acetone for 10 min at room temperature, and then reacted with anti-SEOV polyclonal antiserum (rabbit), or sera from vaccinated animals. Antibodies were diluted in PBS containing 3% fetal bovine serum (FBS) and then incubated on transfected cells for 30 min at 37°C. Cover slips were rinsed three times with PBS and incubated for 30 min at 37°C with biotinylated donkey anti-rabbit followed by streptavidin conjugated fluorescein (Amersham), or fluorescein-labeled goat anti-hamster antibody (Kirkegaard & Perry Laboratories) diluted in PBS,3% FBS. Evans blue (Fisher) was included in the secondary antibody solution as a counter stain. Cover slips were rinsed three times with PBS and once with deionized water and then were placed on a drop of fluorescence mounting medium (DAKO) on glass slides. Cells were observed with a Zeiss Axioplan fluorescence microscope.

### Immunoprecipitation

COS cells grown in T-25 cell culture flasks were transfected with 8 µg of plasmid DNA using Fugene6 (Boehringer Mannheim). After 24 hr, expression products were radiolabeled with Promix ([³⁵S]-methionine and [³⁵S]-cysteine, Amersham) and immunoprecipitated as described previously (Schmaljohn *et al*., 1997, supra).

### Vaccination with the gene gun

Cartridges for the gene gun were prepared as described previously (Eisenbraun *et al.,* 1993, *DNA Cell. Biol.* 12, 791-797; Schmaljohn *et al*. 1997, supra). Briefly, ~1.5 µg of DNA was precipitated onto 0.5 mg of ~ 2 µm diameter gold beads (Degussa). DNA-coated gold beads were used to coat the inner surface of Tefzel tubing (McMaster-Carr). Cartridges were made by cutting the DNA-gold-coated Tefzel tubing into ~0.5-inch sections, each containing -0.5 mg of gold, coated with ~0.75 µg of DNA (~50% of the precipitated DNA bound to the gold as determined by elution and fluorometric assay). To vaccinate animals, abdominal fur was removed with clippers and DNA-coated gold was administered to nonoverlapping sites on the abdominal epidermis by using the gene gun (Powderject-XR delivery device, Powderject Vaccines, Inc.) as described previously (Pertmer *et al.,* 1995, *Vaccine* 13, 1427-1430). BALB/c mice (National Cancer Institute) and outbred Syrian hamsters (Charles River) were vaccinated with two or four cartridges, respectively, under 400 p.s.i. of helium pressure. This research was conducted in accordance with procedures described in the Guide for the Care and Use of Laboratory Animals (National Institute of Health, 1996). The facilities are fully accredited by the American Association for Accreditation of Laboratory Animal Care.

### Vaccination by intramuscular needle inoculation

Plasmid DNA (25 µg) suspended in 50 µl of PBS (pH 7.4) was injected into the gastrocnemius muscle of anesthetized mice with a 28.5-gauge needle.

### ELISA

To detect SEOV G1 and G2, ELISA plates (Costar) were coated with SEOV-infected Vero E6 cell lysates [⁻Co-irradiated (3 million rads) to inactivate virus] diluted in carbonate buffer (pH. 9.6) overnight at 4°C. Plates were blocked with PBS, 3% skim milk, 0.05% Tween-20 (blocking solution) for 1 hr at 37°C and then washed once with PBS, 0.05% Tween-20 (wash solution). Mouse or hamster sera diluted in blocking solution were added to the wells, and plates were incubated as before. Plates were washed four times with wash solution and then incubated for 1 hr with horseradish peroxidase (HRP)-conjugated goat antimouse IgG (Cat. no. A-4416, Sigma) at a dilution of 1/1,000 or peroxidase-labeled goat anti-hamster antibody (Cat. no. 14-22-06, Kirkegaard & Perry Laboratories) diluted 1/2,000 in blocking solution. Plates were washed as before and then incubated for 10 min at room temperature with tetra-methylbenzidine substrate (TMB, Cat. no. 50-76-04, Kirkegaard & Perry Laboratories). The colorimetric reaction was stopped by adding Stop solution (Cat. no. 50-85-04, Kirkegaard & Perry Laboratories) and the optical density (O.D.), 450 nm, was determined. The method for detecting SEOV N-specific antibodies was adapted from a previously described technique (Elgh *et al.,* 1997, *J. Clin. Microbiol*. 35, 1122-1130). Amino acids 1-117 of the nucleocapsid protein of SEOV (strain SR-11) were expressed as a histidine-tagged fusion protein by using the pRSET plasmid (Invitrogen) in *Escherichia coli* BL21 (DE3) (Novagen, Inc.) and purified by affinity chromatography on Ni-NTA columns (Qiagen). Antigen was diluted in carbonate buffer (pH 9.6), added to 96-well microtiter plates (Maxisorp; NUNC) (0.2 µg in 100 µl per well), and incubated overnight at 4°C. Plates were washed in deionized water and incubated with blocking solution for 30 min at room temperature. After plates were washed in deionized water, 100 µl of serum (diluted in blocking buffer containing *E. coli* antigen extract [0.6 mg/ml]) was added to duplicate wells. Plates were incubated at 37°C for 1 h and again washed as before. Addition of HRP secondary antibody and colorimetric detection were added as described above for the SEOV G1-G2 ELISA. End-point titers were determined as the highest dilution with an O.D. greater than the mean O.D. value of serum samples from negative control plasmid (pWRG7077)-vaccinated animals plus three standard deviations.

### Plaque reduction neutralization tests (PRNT)

Neutralization assays were performed essentially as previously described (Chu *et al.,* 1995, *J. Virol*. 69, 6417-6423). Serum samples were diluted in cEMEM and then combined with an equal volume (111 µl) of cEMEM containing ~75 PFU of SEOV. This serum-virus mixture was incubated overnight at 4°C and then 200 µl/well was added to 6well plates containing Vero E6 monolayers 3-7 days old. After a 1-hr adsorption at 37°C, 3 ml of overlay medium (Earl's basal minimal essential medium (EBME), 10 mM HEPEs, 0.6% agarose [Sea Kem ME agarose], 8 mM L-glutamine, antibiotics) containing 10% FBS and 1x nonessential amino acid mixture (GIBCO BRL) was added to each well. Plates were incubated at 37°C for 7 days and then stained by adding 2 ml/well of overlay medium containing 5% FBS and 5% neutral red solution (GIBCO BRL). Plaques were counted after 2 days at 37°C. Serum samples were heat inactivated (56°C, 30 min) before assay. In experiments where complement was used, serum and virus were incubated overnight in the presence of 5% guinea pig complement (Cat. no. ACL-4051, Accurate Chemical and Scientific Corporation).

### Challenge with SEOV

Syrian hamsters were injected intramuscularly (caudal thigh) with a 25-gauge needle with 10³ PFU of SEOV diluted in 0.2 ml sterile PBS (pH 7.4). The 10³ PFU dose was based on previously pulished work that reported 100% infection of hamsters after this challenge dose and route (Schmaljohn *et al*., 1990, supra; Chu *et al.*, 1995, supra). Twenty-eight days after challenge, the hamsters were anesthetized and exsanguinated by cardiac puncture. Postchallenge serum was evaluated for the presence of anti-SEOV antibody by IFAT, ELISA, and PRNT. A dramatic rise in postchallenge antibody titer to viral proteins other than the immunogens indicated that the hamster was infected with SEOV.
**Cross protection assay.** Groups of 4-5 hamsters were vaccinated with either pWRG/SEO M or negative control plasmid (pWRG7077). Vaccinations consisted of four gene gun administrations (~1.5 ug DNA per administration) three times at three week intervals. Three weeks after the final vaccination, prechallenge serum samples were obtained and the hamsters were challenged with 1,000 PFU of Hantaan virus, 1,000 PFU of Dobrava virus, or 1,000-2,000 PFU of Puumala virus. Twenty-eight days after challenge postchallenge serum samples were obtained. Pre and postchallenge serum samples were evaluated by anti-N ELISA to detect antibody to nucleocapsid, by a Hantaan, Dobrava, or Puumula plaque reduction neutralization test (PRNT) to detect the resective neutralizing antibodies depending on the challenge virus. Details of vaccination procedures, anti-N ELISA, and PRNT are as described in Hooper et al, Virology, March 15,1999.

### EXAMPLE 1

### Transient expression of hantavirus genes.

The cDNA representing the M (SEQ ID NO:1) or S (SEQ ID NO:2) gene segment of the SR-11 strain of SEOV was subcloned into the naked DNA expression vector pWRG7077 downstream of the cytomegalovirus immediate early promoter to yield pWRG/SEO-M (SEQ ID NO:3) and pWRG/SEO-S (SEQ ID NO:4), respectively (Fig. 1). To assess expression by the M and S constructs, these plasmids were transfected into COS cells and the expressed proteins were either immunostained or immunoprecipitated. The presence of stained cells in transfected monolayers incubated with anti-SEOV polyclonal sera indicated that SEOV reactive protein(s) were expressed from both pWRG/SEO-M and pWRG/SEO-S (Fig. 2A). Radiolabeled expression products were immunoprecipitated with anti-SEOV polyclonal antisera and analyzed by SDS-PAGE (Fig. 2B). Immunoprecipitated proteins had apparent molecular sizes expected for G1, G2, and N (80 kDa, 56 kDa, and 48 kDa)(Elliot *et al.* 1984, *J. Gen. Virol*. 65, 1285-1293; Schmaljohn *et al.* 1986, *Virology* 155, 633-643). Additional protein bands with apparent molecular masses of ~37 kDa and ~28 kDa were coprecipitated with N. These polypeptides were immunoprecipitated by a N amino-terminus-specific monoclonal antibody, and could be detected by Western blot, suggesting they represent truncated forms of N (data not shown).

### EXAMPLE 2

**Vaccination of mice with pWRG/SEO-M or pWRG/SEO-S.** To determine if either the M or S construct could elicit an antibody response in mice, groups of 10 6- to 8-week-old female BALB/c mice were vaccinated with pWRG/SEO-M, pWRG/SEO-S, or negative control DNA (pWRG7077) by two different routes: inoculation of the epidermis by gene gun, or inoculation of the gastrocnemius muscle by needle injection. Mice received a priming vaccination followed by two boosts at 4 week intervals. Blood samples collected before each vaccination and 3 weeks after the final boost were screened for a SEOV-specific antibody response by ELISA. Both the M and S constructs elicited a SEOV-specific antibody response by both routes of DNA administration (Fig. 3). Most mice vaccinated with SEOV S by gene gun exhibited specific antibodies after only one vaccination, and the antibody responses increased after subsequent vaccinations (Fig. 3B). To compare the relative antibody responses, end-point ELISA titers were determined for the final blood samples (Table 1). Gene gun vaccination resulted in higher seroconversion frequencies and geometric mean titers (GMT) than intramuscular vaccination with either construct.

**Table 1 : ELISA Titers in Mice Vaccinated with SEOV Naked DNA Vaccines**

| Inoculated DNA | Vaccine route^{a} | Positive/ total | End point titer range | GMT^{b} |
|---|---|---|---|---|
| pWRG/SEO-M | g.g. | 9/10 | 200-1600 | 303 |
| pWRG/SEO-M | i.m. | 7/10 | 100-400 | 132 |
| pWRG/SEO-S | g.g. | 10/10 | 6400-12800 | 7352 |
| pWRG/SEO-S | i.m. | 9/10 | 800-6400 | 1213 |

| | | | | |
|---|---|---|---|---|
| Note. Mice were vaccinated three times at 4-week intervals. Titers were determined for sera collected three weeks after the final boost. | | | | |
| ^{a}g.g. indicates gene gun; i.m., intramuscular needle inoculation. | | | | |
| ^{b}Geometric mean titer of all animals in group. | | | | |

To determine if naked DNA vaccination elicited neutralizing antibodies, we performed plaque reduction neutralization tests (PRNT) on sera collected 3 weeks after the final boost. All (10/10) mice that were vaccinated with pWRG/SEO-M by gene gun exhibited PRNT_{50%} titers ranging from 40 to 1280 (Fig. 4). Only 1/10 mice vaccinated with pWRG/SEO-M by needle injection exhibited a detectable neutralizing antibody response (PRNT_{50%} = 40). Mice vaccinated with pWRG/SEO-S or pWRG7077 by either gene gun or needle inoculation did not develop neutralizing antibodies (Fig. 4).

### EXAMPLE 3

**Vaccination and challenge of hamsters. To** study the protective efficacy of the naked DNA hantavirus vaccines, groups of five hamsters were vaccinated with either pWRG/SEO-M, pWRG/SEO-S, or negative control plasmid pWRG7077 by gene gun (a priming vaccination followed by two boosts at 4-week intervals) and then challenged with SEOV 6 weeks after the final boost. IFAT, ELISA, and PRNT were performed on serum samples taken before vaccination, on the day of challenge, and 4 weeks after challenge. Because there are no disease models for hantavirus in adult rodents, we evaluated whether or not our DNA vaccines could protect against infection rather than prevent disease.

IFAT and ELISA results demonstrated that before vaccination, none of the hamsters was positive for either SEOV G1-G2 or N protein (data not shown). After vaccination (prechallenge sera drawn on day of challenge), five of five hamsters vaccinated with pWRG/SEO-M developed anti-G1-G2 antibodies, and four of five hamsters vaccinated with the pWRG/SEO-S exhibited anti-N antibodies by IFAT (Table 2). The prechallenge ELISA results detected similar antibody responses except that only four of five pWRG/SEO-M vaccinated hamsters were positive in the anti-SEOV ELISA. The pWRG7077 vaccinated hamsters failed to develop both G1-G2- and N-specific antibodies by IFAT and ELISA before challenge.

After challenge, serum samples from hamsters previously vaccinated with pWRG/SEO-M remained negative for anti-nucleocapsid antibody by IFAT indicating the animals were not infected with SEOV. In contrast, postchallenge serum samples from pWRG/SEO-S and pWRG7077 vaccinated hamsters were positive for both G1-G2 and N antibodies, indicating that the animals were infected when challenged with SEOV. Postchallenge ELISA results were similar to the IFAT results; however, three of five of the pWRG/SEO-M vaccinated hamsters exhibited a weak anti-N response after challenge indicating that, although the IFAT were negative, the hamsters had developed antibodies to the challenge virus.

Before vaccination, none of the hamsters had SEOV neutralizing antibody titers (data not shown). After vaccination, all hamsters vaccinated with pWRG/SEO-M developed neutralizing antibodies with PRNT_{80%} titers ranging from 160 to 640 (Table 2). Neutralizing antibody was not detected in pWRG/SEO-S and pWRG7077 prechallenge sera from vaccinated hamsters. After SEOV challenge, neutralizing antibody titers of the pWRG/SEO-M immunized animals remained essentially the same as before challenge (plus or minus twofold). In contrast, the pWRG7077 and pWRG/SEO-S postchallenge neutralizing antibody titers increased significantly from <20 to between 1,280 and >81,920.

**Table 2. Serological responses and protection of hamsters vaccinated with naked DNA vaccines.**

| | | | IFAT_{b} | | | | | |
|---|---|---|---|---|---|---|---|---|
| | | anti-N | | anti-G1-G2 | | PRNT_{50%c} | | |
| Vaccineₐ | Hamster | | | | | | | Protection |
| | | pre | post | pre | post | pre | post | |
| pWRG/SEO-M | 1 | - | - | + | + | 320(20) | 640 | yes |
| | 2 | - | - | + | + | 320(40) | 640 | yes |
| | 3 | - | - | + | + | 320(20) | 1280 | yes |
| | 4 | - | - | + | + | 80(<20) | 160 | yes |
| | 5 | - | - | + | + | 640(20) | 1280 | yes |
| PWRG/SEO-S | 1 | + | + | - | + | <20(<20) | >640 | no |
| | 2 | + | + | - | + | <20(<20) | >640 | no |
| | 3 | + | + | - | + | <20(<20) | >640 | no |
| | 4 | + | + | - | + | <20(<20) | >640 | no |
| | 5 | - | + | - | + | <20(<20) | >640 | no |
| PWRG7077 | 1 | - | + | - | + | <20(<20) | >640 | no |
| | 2 | - | + | - | + | <20(<20) | >640 | no |
| | 3 | - | + | - | + | <20(<20) | >640 | no |
| | 4 | - | + | - | + | <20(<20) | >640 | no |
| | 5 | - | + | - | + | <20(<20) | >640 | no |

| | | | | | | | | |
|---|---|---|---|---|---|---|---|---|
| ^{a} Three vaccinations consisting of 4 gene gun cartridges (about 1.5 ug/cartridge) administered at 4 week intervals. | | | | | | | | |
| ^{b} Sera were screened at 1:200 in IFAT sing pWRG/SEO-S or pWRG/SEO-M trasnfected cells as target antigen for anti-n and anti-G1-G2, respectively. - indicates no reactivity; + indicates reactivity. | | | | | | | | |
| c PRNT values are reciprocal serum dilutions reducing plaque number by 50%. Assays were performed in the presence of 5% guinea pig complement. Numbers in parenthesis are PRNT values of heated serum in the absence of complement. pre, prechallenge serum samples from day of challenge; post, poschallenge serum samples from 4 weeks post challenge. | | | | | | | | |

During the course of this study, we observed that PRNT titers decreased dramatically if sera were heat-inactivated (56°C, 30 min). To test the possibility that the neutralizing activity was complement-enhanced, we performed PRNT assays in the presence of 5% guinea pig complement and found that adding complement increased the PRNT titers of heated serum 4- to 32-fold (Table 2). Complement alone had no effect on SEOV plaque number (data not shown).

### Example 4

### Vaccination with pWRG/SEO-M cross-protects against infection with Hantaan virus

Hamsters vaccinated with pWRG/SEO-M had no detectable anti-N antibody prior to challenge, and the postchallenge anti-N response was undetectable or very low (<= 1:200) (Fig. 6). In contrast, hamsters vaccinated with the negative control plasmid exhibited a drammatic rise in anti-N antibody (~2 log increase over background). The PRNT indicated that vaccination with pWRG/SEO-M elicited detectable levels of cross neutralizing antibodies in 3 of 4 hamsters. The PRNT titers of these hamsters did not rise after challenge. In the one hamster with undetectable (<1:20) Hantaan neutralizing antibody, the PRNT titer did rise; however, the increase was at least 32-fold lower than that seen in the hamsters vaccinated with the negative control plasmid.

The low or absent increase in antibody titers to Hantaan structural proteins (as measured by ELISA and PRNT) after challenge with Hantaan virus indicates that vaccination with the Seoul virus M gene (pWRG/SEO-M) confers protection against infection with Hantaan virus.

### Example 5

### Vaccination with pWRG/SEO-M cross-protects against infection with Dobrava virus.

Hamsters vaccinated with pWRG/SEO-M had no detectable anti-N antibody prior to challenge, and the postchallenge anti-N response was undetectable. In contrast, all but one of the hamsters vaccinated with the negative control plasmid exhibited a drammatic rise in anti-N antibody (postchallenge anti-N titers of between 1:200 and 1:204,800). The PRNT indicated that vaccination with pWRG/SEO-M elicited low levels of cross neutralizing antibodies in 5 of 5 hamsters. The PRNT titers of these hamsters did not rise above 1:80 after challenge. In contrast, all but one hamster vaccinated with the negative control plasmid exhibited a high postchallenge PRNT (1:5120) indicating the hamsters were infected with Dobrava virus. It remains unclear why animal #182 was not infected.

The low or absent increase in antibody titers to Dobrava structural proteins (as measured by ELISA and PRNT) after challenge with Dobrava virus indicates that vaccination with the Seoul virus M gene (pWRG/SEO-M) confers protection against infection with Dobrava virus.

### Example 5

### vaccination with pWRG/SEO-M fails to cross-protect against infection with Puumala virus.

Hamsters vaccinated with pWRG/SEO-M had no detectable anti-N antibody prior to challenge. After challenge, all but one hamster had postchallenge anti-N response greater than or equal to 1:200 indicating the hamsters were infected with Puumala virus. The negative control hamsters had similar pre and postchallenge anti-N titers. The PRNT resulsts indicated that, although vaccination with pWRG/SEO-M elicited neutralizing antibodies against Seoul virus (empty circles), these antibodies did not cross-neutralizing Puumala virus, and failed to protect against infection. The postchallenge Puumala virus PRNT titers of the pWRG/SEO-M vaccinated and negative control vaccinated hamsters were all similar (greater than or equal to 1:160), exept one negative control hamster that was not infected. It remains unclear why negative control animal #220 was not infected.

The increase in antibody titers to Puumala virus structural proteins (as measured by ELISA and PRNT) after challenge with Puumala virus indicates that vaccination with the Seoul virus M gene (pWRG/SEO-M) fails to confers protection against infection with Puumala virus. It should be noted that the hamster with the highest prechallenge anti-Seoul virus PRNT titer was 1:640 (animal #209). It is possible that animals with higher prechallenge neutralizing antibody titers might be cross-protected against Puumala virus.

### DISCUSSION

We report that naked DNA shows promise as a way to vaccinate against hantaviruses. Our results demonstrate that vaccination with cDNA representing either the SEOV M or S genome segment elicited antibody responses in BALB/c mice and Syrian hamsters. Hamsters vaccinated with DNA expressing the M segment, but not S segment, developed neutralizing antibodies and were protected against infection with SEOV.

In our initial immunogenicity experiments, gene gun vaccination of BALB/c mice resulted in higher seroconversion rates than intramuscular needle inoculation for both pWRG/SEO-M and pWRG/SEO-S, as measured by ELISA and PRNT. These preliminary data prompted us to focus on the gene gun rather than the intramuscular route of DNA delivery. However, because only one intramuscular DNA dose was tested (25 µg per animal), it is possible that the seroconversion rate and/or titers could be increased by adjusting parameters such as the amount of DNA injected, site of injection, or vaccination schedule. It is also possible that needle inoculation elicited a predominantly cell-mediated rather than humoral response to the DNA vaccination, as has been seen by others (Feltquate *et al.,* 1997, *J. Immunol*. 158, 2278-2284; Robinson and Torres, 1997, *Semin. Immunol*. 9, 271-283; Gregoriadis, 1998, *Pharm. Res.* 15, 661-670).

Gene gun vaccination with pWRG/SEO-M protected all hamsters against infection with SEOV as measured by 1) undetectable, or barely detectable, postchallenge anti-N antibody response and, 2) PRNT titers that remained essentially unchanged after SEOV challenge. Although the IFAT results indicated that none of the pWRG/SEO-M vaccinated hamsters developed anti-N antibodies after challenge, the more sensitive anti-N ELISA revealed that three hamsters had a weak anti-N antibody response. Because the postchallenge anti-N ELISA GMT of these hamsters (GMT = 50) was more than 50 times lower than the negative control pWRG7077 vaccinated hamsters (GMT > 3,195), it is likely the anti-N antibodies represent an antibody response to either the input SEOV antigen (no replication), or to a limited infection (e.g., one round of replication). The PRNT data provided additional evidence that vaccination with pWRG/SEO-M, but not pWRG/SEO-S, protected hamsters from SEOV infection. Whereas the negative control pWRG7077-vaccinated hamsters' PRNT titers increased from <20 to between 10,280 and 81,920 after challenge, the pWRG/SEO-M-vaccinated hamster PRNT titers remained within twofold of prechallenge levels. Moreover, the postchallenge PRNT titers of the pWRG/SEO-M-vaccinated hamsters (GMT = 368) remained well below the postchallenge PRNT titers of the pWRG7077-vaccinated hamsters (GMT > 27,024). Hamsters vaccinated with pWRG/SEO-S were infected with SEOV after challenge. The pWRG/SEO-S-vaccinated hamsters' postchallenge PRNT titers increased from <20 to between 2,560 and 20,480. Although the postchallenge PRNT titers of the pWRG/SEO-S were high (GMT = 5,885), it is noteworthy that these titers were approximately fivefold lower than the negative control (pWRG7077) postchallenge titers (GMT > 27,024).

The absence of a neutralizing antibody response in mice and hamsters vaccinated with pWRG/SEO-S, with or without complement, is consistent with published data that monoclonal antibodies to G1 and G2, but not N, have neutralizing activity (Dantas *et al.,* 1986, *Virology* 151, 379-384; Arikawa *et al*., 1989, *J. Gen. Virol*. 70, 615-624; Schmaljohn *et al.,* 1990, J. Virol. 64, 3162-3170; Arikawa *et al.,* 1992, *Arch. Virol*. 70, 615-624), and that vaccination with vaccinia recombinants expressing G1 and/or G2, but not N, elicit a neutralizing response (Pensiero *et al.,* 1988, *J. Virol*. 62, 696-702; Schmaljohn *et al*. 1992, *Vaccine* 10, 10-13; Xu *et al.,* 1992, *Am. J. Trop. Med. Hyg*. 47, 397-404; Chu *et al.,* 1995, supra).

Failure of an anti-N immune response to protect against infection was unexpected because others found that vaccination with recombinant N expressed in insect cells, bacteria, or as chimeric HBV core particles, protect against infection (Schmaljohn *et al*., 1990, supra; Yoshimatsu *et al*., 1993, *Arch. Virol.* 130, 365-376; Lundkvist *et al.,* 1996, *Virology* 216, 397-406; Ulrich *et al.,* 1998, *Vaccine* 16, 272-280). Also, vaccination with recombinant vaccinia viruses expressing the SEOV S segment partially protected gerbils from SEOV infection (Xu *et al*. 1992, supra). Failure of naked DNA vaccination with the S segment to protect hamsters in our experiments may be related to a quantitative rather than qualitative deficiency. Although four of five hamsters vaccinated with pWRG/SEO-S developed a detectable antibody response by IFAT and ELISA, the titers were relatively low (ELISA titers less than or equal to 1:1,600). The reason for these low anti-N antibody titers in hamsters, but not mice, remains unclear. Fault cannot lie with the gene gun cartridges as the same lot was used to vaccinate both hamsters and mice on the same day; and the mouse seroconversion rate was 90%, with an ELISA GMT of over 7,000. Low antibody titers may reflect a poor overall immune response to vaccination with pWRG/SEO-S in hamsters or, alternatively, it is possible that epidermal administration of S DNA with the gene gun results in a predominantly cell-mediated, rather than humoral, response to N. Nevertheless, our findings demonstrate that there was a detectable anti-N immune response in four of five hamsters after naked DNA gene gun vaccination with pWRG/SEO-S, and that this response, whether humoral and/or cell-mediated, was insufficient to protect against SEOV infection. The -fivefold difference in postchallenge PRNT titers of pWRG/SEO-S versus pWRG7077 vaccinated hamsters suggests pWRG/SEO-S vaccination may nonetheless limit SEOV infection.

We found that SEOV neutralizing activity elicited in mice and hamsters was reduced 4- to 32-fold if the sera was heat inactivated (56°C, 30 min). This loss in neutralizing activity could be completely reversed if guinea pig complement was included in the PRNT. These data are consistent with those of others who reported that hantavirus neutralizing activity in sera from humans, mice, and especially rats, was enhanced by complement (Takenaka *et al*., 1985, *Arch. Virol*. 84, 197-206; Asada *et al.,* 1989, *J. Gen. Virol.* 70, 819-825). The observation that complement plays a major role in hantavirus neutralization is notable because PRNT titers are commonly used to assess the immunogenicity and predicted efficacy of hantavirus vaccines. Conditions that deplete or add complement, such as heat inactivation or addition of exogenous complement (e.g., as a purified protein fraction or as fresh animal sera), could alter PRNT titers and, therefore, could alter the assessment of candidate vaccines.

Future studies are aimed at developing naked DNA vaccines that protect against other HFRS-associated hantaviruses, including HTNV, DOBV, and PUUV; and also against the recently discovered North American hantaviruses associated with hantavirus pulmonary syndrome (reviewed in Schmaljohn and Hjelle, 1997, *Emerg. Infect. Dis.* 3, 95-104). We are also interested in evaluating the protective efficacy of candidate vaccines against increasing challenge doses, as well as different routes of challenge.
Schmaljohn and Hooper
Title: DNA Vaccines Against Hantavirus Infections
SEQ ID NO: 3 : pWRG/SEO-M 
GGGGGGGGGGGGCGCTGAGGTCTGCCTCGTGAAGAAGGTGTTGCTGACTCATACCAGGCCT
GAATCGCCCCATCATCCAG
CCAGAAAGTGAGGGAGCCACGGTTGATGAGAGCTTTGTTGTAGGTGGACCAGTTGGTGATT
TTGAACTTTTGCTTTGCCA
CGGAACGGTCTGCGTTGTCGGGAAGATGCGTGATCTGATCCTTCAACTCAGCAAAAGTTCG
ATTTATTCAACAAAGCCGC
CGTCCCGTCAAGTCAGCGTAATGCTCTGCCAGTGTTACAACCAATTAACCAATTCTGATTA
GAAAAACTCATCGAGCATC
AAATGAAACTGCAATTTATTCATATCAGGATTATCAATACCATATTTTTGAAAAAGCCGTT
TCTGTAATGAAGGAGAAAA
CTCACCGAGGCAGTTCCATAGGATGGCAAGATCCTGGTATCGGTCTGCGATTCCGACTCGT
CCAACATCAATACAACCTA
TTAATTTCCCCTCGTCAAAAATAAGGTTATCAAGTGAGAAATCACCATGAGTGACGACTGA
ATCCGGTGAGAATGGCAAA
AGCTTATGCATTTCTTTCCAGACTTGTTCAACAGGCCAGCCATTACGCTCGTCATCAAAAT
CACTCGCATCAACCAAACC
GTTATTCATTCGTGATTGCGCCTGAGCGAGACGAAATACGCGATCGCTGTTAAAAGGACAA
TTACAAACAGGAATCGAAT
GCAACCGGCGCAGGAACACTGCCAGCGCATCAACAATATTTTCACCTGAATCAGGATATTC
TTCTAATACCTGGAATGCT
GTTTTCCCGGGGATCGCAGTGGTGAGTAACCATGCATCATCAGGAGTACGGATAAAATGCT
TGATGGTCGGAAGAGGCAT
AAATTCCGTCAGCCAGTTTAGTCTGACCATCTCATCTGTAACATCATTGGCAACGCTACCT
TTGCCATGTTTCAGAAACA
ACTCTGGCGCATCGGGCTTCCCATACAATCGATAGATTGTCGCACCTGATTGCCCGACATT
ATCGCGAGCCCATTTATAC
CCATATAAATCAGCATCCATGTTGGAATTTAATCGCGGCCTCGAGCAAGACGTTTCCCGTT
GAATATGGCTCATAACACC
CCTTGTATTACTGTTTATGTAAGCAGACAGTTTTATTGTTCATGATGATATATTTTTATCT
TGTGCAATGTAACATCAGA
GATTTTGAGACACAACGTGGCTTTCCCCCCCCCCCCGGCATGCCTGCAGGTCGACATAAAT
CAATATTGGCTATTGGCCA
TTGCATACGTTGTATCTATATCATAATATGTACATTTATATTGGCTCATGTCCAATATGAC
CGCCATGTTGACATTGATT
ATTGACTAGTTATTAATAGTAATCAATTACGGGGTCATTAGTTCATAGCCCATATATGGAG
TTCCGCGTTACATAACTTA
CGGTAAATGGCCCGCCTCGTGACCGCCCAACGACCCCCGCCCATTGACGTCAATAATGACG
TATGTTCCCATAGTAACGC
CAATAGGGACTTTCCATTGACGTCAATGGGTGGAGTATTTACGGTAAACTGCCCACTTGGC
AGTACATCAAGTGTATCAT
ATGCCAAGTCCGGCCCCCTATTGACGTCAATGACGGTAAATGGCCCGCCTGGCATTATGCC
CAGTACATGACCTTACGGG
ACTTTCCTACTTGGCAGTACATCTACGTATTAGTCATCGCTATTACCATGGTGATGCGGTT
TTGGCAGTACACCAATGGG
CGTGGATAGCGGTTTGACTCACGGGGATTTCCAAGTCTCCACCCCATTGACGTCAATGGGA
GTTTGTTTTGGCACCAAAA
TCAACGGGACTTTCCAAAATGTCGTAATAACCCCGCCCCGTTGACGCAAATGGGCGGTAGG
CGTGTACGGTGGGAGGTCT
 
ATATAAGCAGAGCTCGTTTAGTGAACCGTCAGATCGCCTGGAGACGCCATCCACGCTGTTT
TGACCTCCATAGAAGACAC
CGGGACCGATCCAGCCTCCGCGGCCGGGAACGGTGCATTGGAACGCGGATTCCCCGTGCCA
AGAGTGACGTAAGTACCGC
CTATAGACTCTATAGGCACACCCCTTTGGCTCTTATGCATGCTATACTGTTTTTGGCTTGG
GGCCTATACACCCCCGCTC
CTTATGCTATAGGTGATGGTATAGCTTAGCCTATAGGTGTGGGTTATTGACCATTATTGAC
CACTCCCCTATTGGTGACG
ATACTTTCCATTACTAATCCATAACATGGCTCTTTGCCACAACTATCTCTATTGGCTATAT
GCCAATACTCTGTCCTTCA
GAGACTGACACGGACTCTGTATTTTTACAGGATGGGGTCCCATTTATTATTTACAAATTCA
CATATACAACAACGCCGTC
CCCCGTGCCCGCAGTTTTTATTAAACATAGCGTGGGATCTCCACGCGAATCTCGGGTACGT
GTTCCGGACATGGGCTCTT
CTCCGGTAGCGGCGGAGCTTCCACATCCGAGCCCTGGTCCCATGCCTCCAGCGGCTCATGG
TCGCTCGGCAGCTCCTTGC
TCCTAACAGTGGAGGCCAGACTTAGGCACAGCACAATGCCCACCACCACCAGTGTGCCGCA
CAAGGCCGTGGCGGTAGGG
TATGTGTCTGAAAATGAGCTCGGAGATTGGGCTCGCACCGTGACGCAGATGGAAGACTTAA
GGCAGCGGCAGAAGAAGAT
GCAGGCAGCTGAGTTGTTGTATTCTGATAAGAGTCAGAGGTAACTCCCGTTGCGGTGCTGT
TAACGGTGGAGGGCAGTGT
AGTCTGAGCAGTACTCGTTGCTGCCGCGCGCGCCACCAGACATAATAGCTGACAGACTAAC
AGACTGTTCCTTTCCATGG
GTCTTTTCTGCAGTCACCGTCCAAGCTTGCGGCCGCGGATCTGCAGGAATTCGGCACGAGA
GTAGTAGACTCCGCAAGAA
ACAGCAGTTAAAGAACAATAGGATCATGTGGAGTTTGCTATTACTGGCCGCTTTAGTTGGC
CAAGGCTTTGCATTAAAAA
ATGTATTTGACATGAGAATTCAGTTGCCCCACTCAGTCAACTTTGGGGAAACAAGTGTGTC
AGGCTATACAGAATTTCCC
CCACTCTCATTACAGGAGGCAGAACAGCTAGTGCCAGAGAGCTCATGCAACATGGACAACC
ACCAGTCACTCTCAACAAT
AAATAAATTAACCAAGGTCATATGGCGGAAAAAGCAAATCAGGAATCAGCAAACCAGAAT
TCATTTGAAGTTGTGGAAA
GTGAAGTCAGCTTTAAAGGGTTGTGTATGTTAAAGCATAGAATGGTTGAAGAATCATATAG
AAATAGGAGATCAGTAATC
TGTTATGATCTAGCCTGTAATAGTACATTCTGTAAACCAACTGTTTATATGATTGTTCCTA
TACATGCTTGCAACATGAT
GAAAAGCTGTTTGATTGGCCTTGGCCCCTACAGAATCCAGGTTGTCTATGAAAGGACATAC
TGCACTACGGGTATATTGA
CAGAAGGAAAATGCTTTGTCCCTGACAAGGCTGTTGTCAGTGCATTGAAAAGAGGCATGTA
TGCTATAGCAAGCATAGAG
ACAATCTGCTTTTTTATTCATCAGAAAGGAATACATATAAGATAGTGACTGCCATTACAT
CAGCAATGGGCTCCAAATG
TAATAATACAGATACTAAAGTTCAAGGATATTATATCTGTATTATTGGTGGAAACTCCGCC
CCTGTATATGCCCCTGCTG
GTGAAGACTTCAGAGCAATGGAGGTTTTTCTGGGATTATTACATCACCACATGGAGAAGA
CCATGACCTACCCGGCGAA
GAAATCGCAACGTACCAGATTTCAGGGCAGATAGAGGCAAAAATCCCTCATACAGTGAGCT
CCAAAAACTTAAAATTGAC
TGCTTTTGCAGGTATTCCATCATACTCATCAACTAGTATATTGGCTGCTTCAGAAGATGGT
CGTTTCATATTTAGTCCTG
GTTTATTTCCTAACCTAAATCAGTCAGTCTGTGACAACAATGCACTCCCTTTAATCTGGAG
GGGCCTAATTGATTTAACG
 
GGATACTATGAGGCAGTCCACCCTTGCAATGTGTTCTGTGTCTTATCAGGACCAGGTGCTT
CATGTGAGGCCTTTTCAGA
AGGAGGTATTTTCAATATTACTTCTCCAATGTGTCTGGTGTCTAAGCAAAATAGATTTAGA
GCAGCTGAGCAGCAGATTA
GCTTTGTCTGCCAAAGAGTTGATATGGATATTATAGTGTACTGTAATGGTCAGAAAAAAAC
AATCCTAACAAAAACATTA
GTTATAGGCCAATGTATTTATACTATTACAAGTCTCTTTTCACTGTTACCAGGGGTTGCCC
ATTCTATTGCTATTGAGTT
GTGTGTTCCAGGGTTTCATGGCTGGGCCACAGCTGCACTTTTGATTACATTCTGCTTCGGC
TGGGTATTGATTCCTGCAT
GTACATTAGCTATTCTTTTAGTCCTTAAGTTCTTTGCAAATATCCTTCATACAAGCAATCA
AGAGAACCGATTCAAAGCC
ATTCTACGGAAAATAAAGGAGGAGTTTGAAAAAACAAAGGGTTCCATGGTTTGTGAGATCT
GTAAGTATGAGTGTGAAAC
ATTAAAGGAATTGAAGGCACATAACCTATCATGTGTTCAAGGAGAGTGCCCATATTGCTTT
ACCCACTGTGAACCGACAG
AAACTGCAATTCAGGCACATTACAAAGTTTGTCAAGCCACCCACCGATTCAGAGAAGATTT
AAAAAAGACTGTAACTCCT
CAAAATATTGGGCCAGGCTGTTACCGAACACTAAATCTTTTTAGGTATAAAAGTAGGTGTT
ATATTCTGACAATGTGGAC
TCTTCTTCTCATTATTGAATCCATCCTCTGGGCAGCAAGTGCAGCAGAAATCCCCCTTGTC
CCTCTCTGGACAGATAATG
CTCATGGCGTTGGGAGTGTTCCTATGCATACGGATCTTGAATTAGACTTCTCTTTGCCATC
CAGTTCTAAGTACACATAC
AAAAGACATCTCACAAACCCAGTTAATGACCAACAGAGTGTCTCATTGCATATAGAAATTG
AAAGTCAAGGCATTGGTGC
TGCTGTTCATCATCTTGGACATTGGTATGATGCAAGATTGAATCTAAAAACCTCATTTCAT
TGTTATGGTGCCTGCACAA
AATATCAATACCCATGGCACACTGCAAAATGCCATTTTGAGAAAGATTATGAGTATGAAAA
TAGCTGGGCTTGCAACCCC
CCAGATTGCCCAGGGGTTGGTACAGGTTGTACTGCTTGTGGATTATATCTAGATCAATTGA
AGCCGGTAGGAACAGCCTT
TAAAATTATAAGTGTAAGATACAGTAGAAAAGTGTGCGTGCAGTTTGGTGAAGAACACCTT
TGTAAAACAATTGATATGA
ATGATTGCTTTGTGACTAGGCATGCCAAAATATGTATAATTGGGACTGTATCTAAGTTTTC
TCAAGGTGACACTCTACTA
TTTCTGGGGCCCATGGAAGGAGGTGGTATAATCTTTAAACACTGGTGTACATCTACCTGTC
ACTTTGGAGACCCTGGTGA
TGTCATGGGTCCAAAAGATAAACCATTTATTTGCCCTGAATTCCCAGGGCAATTTAGGAAA
AAATGTAACTTTGCCACAA
CTCCAGTTTGTGAATATGATGGAAACATTATATCAGGCTATAAGAAAGTACTTGCAACAAT
TGATTCTTTCCAATCATTT
AACACAAGCAATATACACTTCACTGATGAGAGAATTGAATGGAGAGACCCTGATGGCATGC
TTCGGGATCATATTAATAT
TGTTATTTCTAAAGATATTGATTTTGAAAATTTGGCTGAGAATCCTTGTAAAGTAGGGCTC
CAGGCAGCAAACATAGAAG
GTGCCTGGGGTTCAGGTGTCGGGTTTACACTCACATGCAAGGTGTCTCTCACAGAATGCCC
AACATTTCTTACATCAATA
AAGGCCTGTGACATGGCAATTTGTTATGGTGCAGAAAGTGTGACACTCTCACGAGGACAAA
ATACTGTCAAAATTACCGG
GAAAGGTGGCCATAGTGGTTCTTCATTCAAATGCTGTCATGGGAAAGAATGTTCATCAACT
GGCCTCCAAGCCAGTGCAC
CACATCTGGATAAGGTAAATGGTATCTCTGAGTTAGAAAACGAGAAAGTTTATGATGACGG
TGCACCTGAATGTGGCATT
 
ACTTGTTGGTTTAAAAATCAGGTGAATGGGTTATGGTATAATCAATGGGAACTGGGTTG
TCCTAATTGTCTTGTGTGT
ACTGCTGCTCTTTTCTCTTATCCTGTTGAGCATCTTGTGTCCTGTTAGAAAGCATAAAAA
TCATAAATCCCACCTAACA
ATCTTCACATCATGTATCGATTTTCAAACACTTTATCATTTAGAACTTAACTTGGCACTAC
TATCTGATAACTGACTTTC
ATTTTTATTTTTATATGGATTAATTACTAAAAAAATACTCTCTCGTGCCGAATTCGATAT
CAAGCTTATCGATACCGTC
GACCTCGAGGGGGGGCCCGGTACCCGGATCCTCGCAATCCCTAGGAGGATTAGGCAAGGG
CTTGAGCTCACGCTCTTGT
GAGGGACAGAAATACAATCAGGGGCAGTATATGAATACTCCATGGAGAAACCCAGATCTAC
GTATGATCAGCCTCGACTG
TGCCTTCTAGTTGCCAGCCATCTGTTGTTTGCCCCTCCCCCGTGCCTTCCTTGACCCTGGA
AGGTGCCACTCCCACTGTC
CTTTCCTAATAAAATGAGGAAATTGCATCGCATTGTCTGAGTAGGTGTCATTCTATTCTGG
GGGGTGGGGTGGGGCAGGA
CAGCAAGGGGGAGGATTGGGAAGACAATAGCAGGCATGCTGGGGATGCGGTGGGCTCTATG
GCTTCTGAGGCGGAAAGAA
CCAGCTGGGGCTCGACAGCTCGACTCTAGAATTGCTTCCTCGCTCACTGACTCGCTGCGCT
CGGTCGTTCGGCTGCGGCG
AGCGGTATCAGCTCACTCAAAGGCGGTAATACGGTTATCCACAGAATCAGGGGATAACGCA
GGAAAGAACATGTGAGCAA
AAGGCCAGCAAAAGGCCAGGAACCGTAAAAAGGCCGCGTTGCTGGCGTTTTTCCATAGGCT
CCGCCCCCCTGACGAGCAT
CACAAAAATCGACGCTCAAGTCAGAGGTGGCGAAACCCGACAGGACTATAAAGATACCAGG
CGTTTCCCCCTGGAAGCTC
CCTCGTGCGCTCTCCTGTTCCGACCCTGCCGCTTACCGGATACCTGTCCGCCTTTCTCCCT
TCGGGAAGCGTGGCGCTTT
CTCAATGCTCACGCTGTAGGTATCTCAGTTCGGTGTAGGTCGTTCGCTCCAAGCTGGGCTG
TGTGCACGAACCCCCCGTT
CAGCCCGACCGCTGCGCCTTATCCGGTAACTATCGTCTTGAGTCCAACCCGGTAAGACACG
ACTTATCGCCACTGGCAGC
AGCCACTGGTAACAGGATTAGCAGAGCGAGGTATGTAGGCGGTGCTACAGAGTTCTTGAAG
TGGTGGCCTAACTACGGCT
ACACTAGAAGGACAGTATTTGGTATCTGCGCTCTGCTGAAGCCAGTTACCTTCGGAAAAAG
AGTTGGTAGCTCTTGATCC
GGCAAACAAACCACCGCTGGTAGCGGTGGTTTTTTTGTTTGCAAGCAGCAGATTACGCGCA
GAAAAAAAGGATCTCAAGA
AGATCCTTTGATCTTTTCTACGGGGTCTGACGCTCAGTGGAACGAAAACTCACGTTAAGGG
ATTTTGGTCATGAGATTAT
CAAAAAGGATCTTCACCTAGATCCTTTTAAATTAAAAATGAAGTTTTAAATCAATCTAAAG
TATATATGAGTAAACTTGG
TCTGACAGTTACCAATGCTTAATCAGTGAGGCACCTATCTCAGCGATCTGTCTATTTCGTT
CATCCATAGTTGCCTGACTC
 SEQ ID NO:4: pWRG/SEO-S 
GGGGGGGGGGGGCGCTGAGGTCTGCCTCGTGAAGAAGGTGTTGCTGACTCATACCAGGCCT
GAATCGCCCCATCATCCAG
CCAGAAAGTGAGGGAGCCACGGTTGATGAGAGCTTTGTTGTAGGTGGACCAGTTGGTGATT
TTGAACTTTTGCTTTGCCA
CGGAACGGTCTGCGTTGTCGGGAAGATGCGTGATCTGATCCTTCAACTCAGCAAAAGTTCG
ATTTATTCAACAAAGCCGC
 
CGTCCCGTCAAGTCAGCGTAATGCTCTGCCAGTGTTACAACCAATTAACCAATTCTGATTA
GAAAAACTCATCGAGCATC
AAATGAAACTGCAATTTATTCATATCAGGATTATCAATACCATATTTTTGAAAAAGCCGTT
TCTGTAATGAAGGAGAAAA
CTCACCGAGGCAGTTCCATAGGATGGCAAGATCCTGGTATCGGTCTGCGATTCCGACTCGT
CCAACATCAATACAACCTA
TTAATTTCCCCTCGTCAAAAATAAGGTTATCAAGTGAGAAATCACCATGAGTGACGACTGA
ATCCGGTGAGAATGGCAAA
AGCTTATGCATTTCTTTCCAGACTTGTTCAACAGGCCAGCCATTACGCTCGTCATCAAAAT
CACTCGCATCAACCAAACC
GTTATTCATTCGTGATTGCGCCTGAGCGAGACGAAATACGCGATCGCTGTTAAAAGGACAA
TTACAAACAGGAATCGAAT
GCAACCGGCGCAGGAACACTGCCAGCGCATCAACAATATTTTCACCTGAATCAGGATATTC
TTCTAATACCTGGAATGCT
GTTTTCCCGGGGATCGCAGTGGTGAGTAACCATGCATCATCAGGAGTACGGATAAAATGCT
TGATGGTCGGAAGAGGCAT
AAATTCCGTCAGCCAGTTTAGTCTGACCATCTCATCTGTAACATCATTGGCAACGCTACCT
TTGCCATGTTTCAGAAACA
ACTCTGGCGCATCGGGCTTCCCATACAATCGATAGATTGTCGCACCTGATTGCCCGACATT
ATCGCGAGCCCATTTATAC
CCATATAAATCAGCATCCATGTTGGAATTTAATCGCGGCCTCGAGCAAGACGTTTCCCGTT
GAATATGGCTCATAACACC
CCTTGTATTACTGTTTATGTAAGCAGACAGTTTTATTGTTCATGATGATATATTTTTATCT
TGTGCAATGTAACATCAGA
GATTTTGAGACACAACGTGGCTTTCCCCCCCCCCCCGGCATGCCTGCAGGTCGACATAAAT
CAATATTGGCTATTGGCCA
TTGCATACGTTGTATCTATATCATAATATGTACATTTATATTGGCTCATGTCCAATATGAC
CGCCATGTTGACATTGATT
ATTGACTAGTTATTAATAGTAATCAATTACGGGGTCATTAGTTCATAGCCCATATATGGAG
TTCCGCGTTACATAACTTA
CGGTAAATGGCCCGCCTCGTGACCGCCCAACGACCCCCGCCCATTGACGTCAATAATGACG
TATGTTCCCATAGTAACGC
CAATAGGGACTTTCCATTGACGTCAATGGGTGGAGTATTTACGGTAAACTGCCCACTTGGC
AGTACATCAAGTGTATCAT
ATGCCAAGTCCGGCCCCCTATTGACGTCAATGACGGTAAATGGCCCGCCTGGCATTATGCC
CAGTACATGACCTTACGGG
ACTTTCCTACTTGGCAGTACATCTACGTATTAGTCATCGCTATTACCATGGTGATGCGGTT
TTGGCAGTACACCAATGGG
CGTGGATAGCGGTTTGACTCACGGGGATTTCCAAGTCTCCACCCCATTGACGTCAATGGGA
GTTTGTTTTGGCACCAAAA
TCAACGGGACTTTCCAAAATGTCGTAATAACCCCGCCCCGTTGACGCAAATGGGCGGTAGG
CGTGTACGGTGGGAGGTCT
ATATAAGCAGAGCTCGTTTAGTGAACCGTCAGATCGCCTGGAGACGCCATCCACGCTGTTT
TGACCTCCATAGAAGACAC
CGGGACCGATCCAGCCTCCGCGGCCGGGAACGGTGCATTGGAACGCGGATTCCCCGTGCCA
AGAGTGACGTAAGTACCGC
CTATAGACTCTATAGGCACACCCCTTTGGCTCTTATGCATGCTATACTGTTTTTGGCTTGG
GGCCTATACACCCCCGCTC
CTTATGCTATAGGTGATGGTATAGCTTAGCCTATAGGTGTGGGTTATTGACCATTATTGAC
CACTCCCCTATTGGTGACG
ATACTTTCCATTACTAATCCATAACATGGCTCTTTGCCACAACTATCTCTATTGGCTATAT
GCCAATACTCTGTCCTTCA
GAGACTGACACGGACTCTGTATTTTTACAGGATGGGGTCCCATTTATTATTTACAAATTCA
CATATACAACAACGCCGTC
 
CCCCGTGCCCGCAGTTTTTATTAAACATAGCGTGGATCTCCACGCGAATCTCGGGTACGT
GTTCCGGACATGGGCTCTT
CTCCGGTAGCGGCGGAGCTTCCACATCCGAGCCCTGGTCCCATGCCTCCAGCGGCTCATGG
TCGCTCGGCAGCTCCTTGC
TCCTAACAGTGGAGGCCAGACTTAGGCACAGCACAATGCCCACCACCACCAGTGTGCCGCA
CAAGGCCGTGGCGGTAGGG
TATGTGTCTGAAAATGAGCTCGGAGATTGGGCTCGCACCGTGACGCAGATGGAAGACTTAA
GGCAGCGGCAGAAGAAGAT
GCAGGCAGCTGAGTTGTTGTATTCTGATAAGAGTCAGAGGTAACTCCCGTTGCGGTGCTGT
TAACGGTGGAGGGCAGTGT
AGTCTGAGCAGTACTCGTTGCTGCCGCGCGCCACCAGACATAATAGCTGACAGACTAAC
AGACTGTTCCTTTCCATGG
GTCTTTTCTGCAGTCACCGTCCAAGCTTGCGGCCAATTCGGCACGAGAGAGTAGTAGACTC
CCTAAAGAGCTACTACACT
AACAAGAAAAATGGCAACTATGGAAGAAATCCAGAGAGAAATCAGTGCTCACGAGGGGCAG
CTTGTGATAGCACGCCAGA
AGGTCAAGGATGCAGAAAAGCAGTATGAGAAGGATCCTGATGACTTAAACAAGAGGGCACT
GCATGATCGGGAGAGTGTC
GCAGCTTCAATACAATCAAAAATTGATGAATTGAAGCGCCAACTTGCCGACAGATTGCAGC
AGGGAAGAACATCCGGGCA
GGACCGGGATCCTACAGGGGTAGAGCCAGGTGATCATCTTAAGGAAAGATCAGCACTAAGC
TACGGGAATACACTGGACC
TGAATAGTCTTGACATTGATGAACCTACAGGACAGACAGCTGATTGGCTGACCATAATTGT
CTATCTGACATCATTCGTG
GTCCCGATCATCTTGAAGGCACTGTACATGTTAACAACACGAGGTAGGCAGACTTCAAAGG
ACAACAAGGGGATGAGGAT
CAGATTCAAGGATGACAGCTCATATGAGGATGTCAATGGAATCAGAAAGCCCAAACATCTG
TATGTGTCAATGCCAAACG
CCCAATCCAGCATGAAGGCTGAAGAGATAACACCAGGAAGATTCCGCACTGCAGTATGTGG
ACTATATCCTGCACAGATA
AAGGCAAGGAATATGGTAAGCCCTGTCATGAGTGTAGTTGGGTTCTTGGCACTGGCAAAAG
ACTGGACATCGAGAATTGA
AGAATGGCTCGGTGCACCCTGCAAATTCATGGCGGAGTCTCTTATTGCCGGGAGTTTATCT
GGGAATCCTGTGAATCGTG
ACTATATCAGACAGAGACAAGGTGCACTTGCAGGGATGGAGCCAAAGGAATTTCAAGCCCT
CAGGCAACATTCAAAGGAT
GCTGGATGTACACTAGTTGAACATATTGAGTCACCATCATCAATATGGGTGTTTGCTGGGG
CCCCTGATAGGTGTCCACC
AACATGCTTGTTTGTCGGAGGGATGGCTGAATTAGGTGCCTTCTTTTCTATACTTCAGGAT
ATGAGGAACACAATCATGG
CTTCAAAAACTGTGGGCACAGCTGATGAAAAGCTTCGAAAGAAATCATCATTCTATCAATC
ATACCTCAGACGCACACAA
TCAATGGGAATACAACTGGACCAGAGGATAATTGTTATGTTTATGGTTGCCTGGGAAAGG
AGGCAGTGGACAACTTTCA
TCTCGGTGATGACATGGATCCAGAGCTTCGTAGCCTGGCTCAGATCTTGATTGACCAGAAA
GTGAAGGAAATCTCAAACC
AGGAACCTATGAAATTATAAGTACATAAATATATAATCAATACTAACTATAGGTTAAGAAA
TACTAATCATTAGTTAATA
AGAATATAGATTTATTGAATAATCATATTAAATAATTAGGTAAGTTAACTAGTATTTAGTT
AAGTTAGCTAATTGATTTA
TATGATTGTCACAATTAAATGTAATCATAAGCACAATCACTGCCATGTATAATCACGGGTA
TACGGGTGGTTTTCATATG
GGGAACAGGGTGGGCTTAGGGCCAGGTCACCTTAAGTGACCTTTTTTTGTATATATGGATG
TAGATTTCAATTGATCGAA
 
TACTAATCCTACTGTCCTCTTTTCTTTTCCTTTCTCCTTCTTTACTAACAAACTACCTCGT
GCCGAATTGGCCGCGGATC
CTCGCAATCCCTAGGAGGATTAGGCAAGGGCTTGAGCTCACGCTCTTGTGAGGGACAGAAA
TACAATCAGGGGCAGTATA
TGAATACTCCATGGAGAAACCCAGATCTACGTATGATCAGCCTCGACTGTGCCTTCTAGTT
GCCAGCCATCTGTTGTTTG
CCCCTCCCCCGTGCCTTCCTTGACCCTGGAAGGTGCCACTCCCACTGTCCTTTCCTAATAA
AATGAGGAAATTGCATCGC
ATTGTCTGAGTAGGTGTCATTCTATTCTGGGGGGTGGGGTGGGGCAGGACAGCAAGGGGGA
GGATTGGGAAGACAATAGC
AGGCATGCTGGGGATGCGGTGGGCTCTATGGCTTCTGAGGCGGAAAGAACCAGCTGGGGCT
CGACAGCTCGACTCTAGAA
TTGCTTCCTCGCTCACTGACTCGCTGCGCTCGGTCGTTCGGCTGCGGCGAGCGGTATCAGC
TCACTCAAAGGCGGTAATA
CGGTTATCCACAGAATCAGGGGATAACGCAGGAAAGAACATGTGAGCAAAAGGCCAGCAAA
AGGCCAGGAACCGTAAAAA
GGCCGCGTTGCTGGCGTTTTTCCATAGGCTCCGCCCCCCTGACGAGCATCACAAAAATCGA
CGCTCAAGTCAGAGGTGGC
GAAACCCGACAGGACTATAAAGATACCAGGCGTTTCCCCCTGGAAGCTCCCTCGTGCGCTC
TCCTGTTCCGACCCTGCCG
CTTACCGGATACCTGTCCGCCTTTCTCCCTTCGGGAAGCGTGGCGCTTTCTCAATGCTCAC
GCTGTAGGTATCTCAGTTC
GGTGTAGGTCGTTCGCTCCAAGCTGGGCTGTGTGCACGAACCCCCCGTTCAGCCCGACCGC
TGCGCCTTATCCGGTAACT
ATCGTCTTGAGTCCAACCCGGTAAGACACGACTTATCGCCACTGGCAGCAGCCACTGGTAA
CAGGATTAGCAGAGCGAGG
TATGTAGGCGGTGCTACAGAGTTCTTGAAGTGGTGGCCTAACTACGGCTACACTAGAAGGA
CAGTATTTGGTATCTGCGC
TCTGCTGAAGCCAGTTACCTTCGGAAAAAGAGTTGGTAGCTCTTGATCCGGCAAACAAACC
ACCGCTGGTAGCGGTGGTT
TTTTTGTTTGCAAGCAGCAGATTACGCGCAGAAAAAAAGGATCTCAAGAAGATCCTTTGAT
CTTTTCTACGGGGTCTGAC
GCTCAGTGGAACGAAAACTCACGTTAAGGGATTTTGGTCATGAGATTATCAAAAAGGATCT
TCACCTAGATCCTTTTAAA
TTAAAAATGAAGTTTTAAATCAATCTAAAGTATATATGAGTAAACTTGGTCTGACAGTTAC
CAATGCTTAATCAGTGAGG
CACCTATCTCAGCGATCTGTCTATTTCGTTCATCCATAGTTGCCTGACTC
 SEQ ID NO: 1: Sapporo rat (SR11) seoul M 
TAGTAGTAGACTCCGCAAGAAACAGCAGTTAAAGAACAATAGGATCATGTGGAGTTTGCTA
TTACTGGCCGCTTTAGTTG
GCCAAGGCTTTGCATTAAAAAATGTATTTGACATGAGAATTCAGTTGCCCCACTCAGTCAA
CTTTGGGGAAACAAGTGTG
TCAGGCTATACAGAATTTCCCCCACTCTCATTACAGGAGGCAGAACAGCTAGTGCCAGAGA
GCTCATGCAACATGGACAA
CCACCAGTCACTCTCAACAATAAATAAATTAACCAAGGTCATATGGCGGAAAAAAGCAAAT
CAGGAATCAGCAAACCAGA
ATTCATTTGAAGTTGTGGAAAGTGAAGTCAGCTTTAAAGGGTTGTGTATGTTAAAGCATAG
AATGGTTGAAGAATCATAT
AGAAATAGGAGATCAGTAATCTGTTATGATCTAGCCTGTAATAGTACATTCTGTAAACCAA
CTGTTTATATGATTGTTCC
TATACATGCTTGCAACATGATGAAAAGCTGTTTGATTGGCCTTGGCCCCTACAGAATCCAG
GTTGTCTATGAAAGGACAT
 
ACTGCACTACGGGTATATTGACAGAAGGAAAATGCTTTGTCCCTGACAAGGCTGTTGTCAG
TGCATTGAAAAGAGGCATG
TATGCTATAGCAAGCATAGAGACAATCTGCTTTTTATTCATCAGAAAGGGAATACATATA
AGATAGTGACTGCCATTAC
ATCAGCAATGGGCTCCAAATGTAATAATACAGATACTAAAGTTCAAGGATATTATATCTGT
ATTATTGGTGGAAACTCCG
CCCCTGTATATGCCCCTGCTGGTGAAGACTTCAGAGCAATGGAGGTTTTTCTGGGATTAT
TACATCACCACATGGAGAA
GACCATGACCTACCCGGCGAAGAAATCGCAACGTACCAGATTTCAGGGCAGATAGAGGCAA
AAATCCCTCATACAGTGAG
CTCCAAAAACTTAAAATTGACTGCTTTTGCAGGTATTCCATCATACTCATCAACTAGTATA
TTGGCTGCTTCAGAAGATG
GTCGTTTCATATTTAGTCCTGGTTTATTTCCTAACCTAAATCAGTCAGTCTGTGACAACAA
TGCACTCCCTTTAATCTGG
AGGGGCCTAATTGATTTAACGGGATACTATGAGGCAGTCCACCCTTGCAATGTGTTCTGTG
TCTTATCAGGACCAGGTGC
TTCATGTGAGGCCTTTTCAGAAGGAGGTATTTTCAATATTACTTCTCCAATGTGTCTGGTG
TCTAAGCAAAATAGATTTA
GAGCAGCTGAGCAGCAGATTAGCTTTGTCTGCCAAAGAGTTGATATGGATATTATAGTGTA
CTGTAATGGTCAGAAAAAA
ACAATCCTAACAAAAACATTAGTTATAGGCCAATGTATTTATACTATTACAAGTCTCTTTT
CACTGTTACCAGGGGTTGC
CCATTCTATTGCTATTGAGTTGTGTGTTCCAGGGTTTCATGGCTGGGCCACAGCTGCACTT
TTGATTACATTCTGCTTCG
GCTGGGTATTGATTCCTGCATGTACATTAGCTATTCTTTTAGTCCTTAAGTTCTTTGCAAA
TATCCTTCATACAAGCAAT
CAAGAGAACCGATTCAAAGCCATTCTACGGAAAATAAAGGAGGAGTTTGAAAAAACAAAGG
GTTCCATGGTTTGTGAGAT
CTGTAAGTATGAGTGTGAAACATTAAAGGAATTGAAGGCACATAACCTATCATGTGTTCAA
GGAGAGTGCCCATATTGCT
TTACCCACTGTGAACCGACAGAAACTGCAATTCAGGCACATTACAAAGTTTGTCAAGCCAC
CCACCGATTCAGAGAAGAT
TTAAAAAAGACTGTAACTCCTCAAAATATTGGGCCAGGCTGTTACCGAACACTAAATCTTT
TTAGGTATAAAAGTAGGTG
TTATATTCTGACAATGTGGACTCTTCTTCTCATTATTGAATCCATCCTCTGGGCAGCAAGT
GCAGCAGAAATCCCCCTTG
TCCCTCTCTGGACAGATAATGCTCATGGCGTTGGGAGTGTTCCTATGCATACGGATCTTGA
ATTAGACTTCTCTTTGCCA
TCCAGTTCTAAGTACACATACAAAAGACATCTCACAAACCCAGTTAATGACCAACAGAGTG
TCTCATTGCATATAGAAAT
TGAAAGTCAAGGCATTGGTGCTGCTGTTCATCATCTTGGACATTGGTATGATGCAAGATTG
AATCTAAAAACCTCATTTC
ATTGTTATGGTGCCTGCACAAAATATCAATACCCATGGCACACTGCAAAATGCCATTTTGA
GAAAGATTATGAGTATGAA
AATAGCTGGGCTTGCAACCCCCCAGATTGCCCAGGGGTTGGTACAGGTTGTACTGCTTGTG
GATTATATCTAGATCAATT
GAAGCCGGTAGGAACAGCCTTTAAAATTATAAGTGTAAGATACAGTAGAAAAGTGTGCGTG
CAGTTTGGTGAAGAACACC
TTTGTAAAACAATTGATATGAATGATTGCTTTGTGACTAGGCATGCCAAAATATGTATAAT
TGGGACTGTATCTAAGTTT
TCTCAAGGTGACACTCTACTATTTCTGGGGCCCATGGAAGGAGGTGGTATAATCTTTAAAC
ACTGGTGTACATCTACCTG
TCACTTTGGAGACCCTGGTGATGTCATGGGTCCAAAAGATAAACCATTTATTTGCCCTGAA
TTCCCAGGGCAATTTAGGA
 
AAAAATGTAACTTTGCCACAACTCCAGTTTGTGAATATGATGGAAACATTATATCAGGCTA
TAAGAAAGTACTTGCAACA
ATTGATTCTTTCCAATCATTTAACACAAGCAATATACACTTCACTGATGAGAGAATTGAAT
GGAGAGACCCTGATGGCAT
GCTTCGGGATCATATTAATATTGTTATTTCTAAAGATATTGATTTTGAAAATTTGGCTGAG
AATCCTTGTAAAGTAGGGC
TCCAGGCAGCAAACATAGAAGGTGCCTGGGGTTCAGGTGTCGGGTTTACACTCACATGCAA
GGTGTCTCTCACAGAATGC
CCAACATTTCTTACATCAATAAAGGCCTGTGACATGGCAATTTGTTATGGTGCAGAAAGTG
TGACACTCTCACGAGGACA
AAATACTGTCAAAATTACCGGAAAGGTGGCCATAGTGGTTCTTCATTCAAATGCTGTCAT
GGGAAAGAATGTTCATCAA
CTGGCCTCCAAGCCAGTGCACCACATCTGGATAAGGTAAATGGTATCTCTGAGTTAGAAAA
CGAGAAAGTTTATGATGAC
GGTGCACCTGAATGTGGCATTACTTGTTGGTTTAAAAAATCAGGTGAATGGGTTATGGGTA
TAATCAATGGGAACTGGGT
TGTCCTAATTGTCTTGTGTGTACTGCTGCTCTTTTCTCTTATCCTGTTGAGCATCTTGTGT
CCTGTTAGAAAGCATAAAA
AATCATAAATCCCACCTAACAATCTTCACATCATGTATCGATTTTCAAACACTTTATCATT
TAGAACTTAACTTGGCACT
ACTATCTGATAACTGACTTTCATTTTTATTTTTATATGGATTAATTACTAAAAAAAATACT
CTCTTCTATCTCCCAATCT
TTTATTGATTCACCGGGGTGCTGTCTTGACATCTGGCGGCGTCTACTACTA
 SEQ ID NO:2: Sapporo rat virus (strain SR-11), seoul S

TAGTAGTAGACTCCCTAAAGAGCTACTACACTAACAAGAAAAATGGCAACTATGGAAGAAA
TCCAGAGAGAAATCAGTGC
TCACGAGGGGCAGCTTGTGATAGCACGCCAGAAGGTCAAGGATGCAGAAAAGCAGTATGAG
AAGGATCCTGATGACTTAA
ACAAGAGGGCACTGCATGATCGGGAGAGTGTCGCAGCTTCAATACAATCAAAAATTGATGA
ATTGAAGCGCCAACTTGCC
GACAGATTGCAGCAGGGAAGAACATCCGGGCAGGACCGGATCCTACAGGGGTAGAGCCAG
GTGATCATCTTAAGGAAAG
ATCAGCACTAAGCTACGGAATACACTGGACCTGAATAGTCTTGACATTGATGAACCTACA
GGACAGACAGCTGATTGGC
TGACCATAATTGTCTATCTGACATCATTCGTGGTCCCGATCATCTTGAAGGCACTGTACAT
GTTAACAACACGAGGTAGG
CAGACTTCAAAGGACAACAAGGGGATGAGGATCAGATTCAAGGATGACAGCTCATATGAGG
ATGTCAATGGAATCAGAAA
GCCCAAACATCTGTATGTGTCAATGCCAAACGCCCAATCCAGCATGAAGGCTGAAGAGATA
ACACCAGGAAGATTCCGCA
CTGCAGTATGTGGACTATATCCTGCACAGATAAAGGCAAGGAATATGGTAAGCCCTGTCAT
GAGTGTAGTTGGGTTCTTG
GCACTGGCAAAAGACTGGACATCGAGAATTGAAGAATGGCTCGGTGCACCCTGCAAATTCA
TGGCGGAGTCTCTTATTGC
CGGGAGTTTATCTGGGAATCCTGTGAATCGTGACTATATCAGACAGAGACAAGGTGCACTT
GCAGGGATGGAGCCAAAGG
AATTTCAAGCCCTCAGGCAACATTCAAAGGATGCTGGATGTACACTAGTTGAACATATTGA
GTCACCATCATCAATATGG
GTGTTTGCTGGGGCCCCTGATAGGTGTCCACCAACATGCTTGTTTGTCGGAGGGATGGCTG
AATTAGGTGCCTTCTTTTC

TATACTTCAGGATATGAGGAACACAATCATGGCTTCAAAAACTGTGGCACAGCTGATGAA
AAGCTTCGAAAGAAATCAT
CATTCTATCAATCATACCTCAGACGCACACAATCAATGGGAATACAACTGGACCAGAGGAT
AATTGTTATGTTTATGGTT
GCCTGGGGAAAGGAGGCAGTGGACAACTTTCATCTCGGTGATGACATGGATCCAGAGCTTC
GTAGCCTGGCTCAGATCTT
GATTGACCAGAAAGTGAAGGAAATCTCAAACCAGGAACCTATGAAATTATAAGTACATAAA
TATATAATCAATACTAACT
ATAGGTTAAGAAATACTAATCATTAGTTAATAAGAATATAGATTTATTGAATAATCATATT
AAATAATTAGGTAAGTTAA
CTAGTATTTAGTTAAGTTAGCTAATTGATTTATATGATTGTCACAATTAAATGTAATCATA
AGCACAATCACTGCCATGT
ATAATCACGGGTATACGGGTGGTTTTCATATGGGGAACAGGGTGGCTTAGGGCCAGGTCA
CCTTAAGTGACCTTTTTTT
GTATATATGGATGTAGATTTCAATTGATCGAATACTAATCCTACTGTCCTCTTTTCTTTTC
CTTTCTCCTTCTTTACTAA
CAACAACAAACTACCTCACACCTTAATATATACTACTTTATTAAGTTGTTAAGTTGTGTCT
TTTTGGGGAGTAAGGGAGT
CTACTACTA

## Claims

1. A composition of matter comprising a carrier particle; and a DNA sequence coated onto the carrier particle, the DNA sequence comprising a promoter operative in the cells of a mammal and a protein coding region coding for a determinant of a hantavirus protein.

2. The composition of Claim 1 wherein said protein coding region encodes a protein selected from the group consisting of M gene segment proteins and S gene segment proteins.

3. The composition of Claim 1 wherein said hantavirus is chosen from the group consisting of Seoul virus, Dobrava virus, Pumuula virus, Hantaan virus, Sin Nombre virus, Black Creek Canal virus, Bayou virus, New York virus, Andes virus, and Laguna Negra virus.

4. The composition of Claim 3 wherein said hantavirus is Seoul virus.

5. The composition of Claim 4 wherein the protein coding region comprises SEQ ID NO:1.

6. The composition of Claim 4 wherein the protein coding region comprises SEQ ID NO:2 .

7. The composition of Claim 1, wherein said DNA sequence comprises pWRG-SEO-M.

8. The composition of Claim 1, wherein said DNA sequence comprises pWRG-SEO-S.

9. Use of a composition according to any of Claims 1 to 8 in the preparation of a vaccine against hantavirus infection.

10. Use of a composition according to any of Claims 1 to 8 in the preparation of a vaccine against infection by the said hantavirus.

11. Use of a composition according to any of Claims 1, 4, 5 and 6, wherein said hantavirus is a first hantavirus, in the preparation of a vaccine against infection by a second hantavirus.

12. Use according to Claim 11 wherein the first hantavirus is Seoul virus and the second hantavirus is Dobrava virus or Hantaan virus.

13. A vaccine against hantavirus infection comprising the composition of any of Claims 1 to 8.

14. A multivalent vaccine for protection against infection with more than one hantavirus comprising a composition of matter comprising a carrier particle having one or more DNA sequence coated onto the carrier particle, the DNA sequence comprising a promoter operative in the cells of a mammal and a protein coding region coding for a determinant of a first hantavirus protein said hantavirus selected from the group consisting of SEOV, Dobrava, Pumuula, Hantaan, Sin Nombre virus, Black Creek Canal virus, Bayou virus, New York virus, Andes virus, and Laguna Negra virus.

15. The multivalent vaccine of Claim 14, further comprising a composition comprising a carrier particle having one or more DNA sequence coated onto the carrier particle, the DNA sequence comprising a promoter operative in the cells of a mammal and a protein coding region coding for a determinant of a second hantavirus different from said first hantavirus, said second hantavirus selected from the group consisting of Seoul virus, Dobrava virus, Pumuula virus, Hantaan virus, Sin Nombre virus, Black Creek Canal virus, Bayou virus, New York virus, Andes virus, and Laguna Negra virus.

## Patentansprüche

1. Stoffzusammensetzung, umfassend ein Trägerteilchen und eine DNS-Sequenz, aufgebracht auf das Trägerteilchen, wobei die DNS-Sequenz einen Promotor, operativ in den Zellen eines Säugers, und eine Protein-Codierungsregion, codierend für eine Determinante eines Hantavirus-Proteins, umfaßt.

2. Zusammensetzung nach Anspruch 1, wobei die Protein-Codierungsregion ein Protein codiert, ausgewählt aus der Gruppe, bestehend aus M-Gensegment-Proteinen und S-Gensegment-Proteinen.

3. Zusammensetzung nach Anspruch 1, wobei das Hantavirus aus der Gruppe ausgewählt ist, bestehend aus Seoul-Virus, Dobrava-Virus, Puumala-Virus, Hantaan-Virus, Sin-Nombre-Virus, Black-Creek-Canal-Virus, Bayou-Virus, New-York-Virus, Andes-Virus und Laguna-Negra-Virus.

4. Zusammensetzung nach Anspruch 3, wobei das Hantavirus das Seoul-Virus ist.

5. Zusammensetzung nach Anspruch 4, wobei die Protein-Codierungsregion SEQ ID NO:1 umfaßt.

6. Zusammensetzung nach Anspruch 4, wobei die Protein-Codierungsregion SEQ ID NO:2 umfaßt.

7. Zusammensetzung nach Anspruch 1, wobei die DNS-Sequenz pWRG-SEO-M umfaßt.

8. Zusammensetzung nach Anspruch 1, wobei die DNS-Sequenz pWRG-SEO-S umfaßt.

9. Verwendung einer Zusammensetzung nach einem der Ansprüche 1 bis 8 bei der Herstellung eines Impfstoffes gegen Hantavirus-Infektion.

10. Verwendung einer Zusammensetzung nach einem der Ansprüche 1 bis 8 bei der Herstellung eines Impfstoffes gegen Infektion durch das Hantavirus.

11. Verwendung einer Zusammensetzung nach einem der Ansprüche 1, 4, 5 und 6, wobei das Hantavirus ein erstes Hantavirus ist, bei der Herstellung eines Impfstoffes gegen Infektion durch ein zweites Hantavirus.

12. Verwendung nach Anspruch 11, wobei das erste Hantavirus das Seoul-Virus ist und das zweite Hantavirus das Dobrava-Virus oder Hantaan-Virus ist.

13. Impfstoff gegen Hantavirus-Infektion, umfassend die Zusammensetzung nach einem der Ansprüche 1 bis 8.

14. Multivalenter Impfstoff zum Schutz gegen Infektion mit mehr als einem Hantavirus, umfassend eine Stoffzusammensetzung, umfassend ein Trägerteilchen mit einer oder mehreren DNS-Sequenzen, aufgebracht auf das Trägerteilchen, wobei die DNS-Sequenz einen Promotor, operativ in den Zellen eines Säugers, und eine Protein-Codierungsregion, codierend für eine Determinante eines ersten Hantavirus-Proteins, umfaßt, wobei das Hantavirus aus der Gruppe, bestehend aus SEOV, Dobrava-, Puumala-, Hantaan-, Sin-Nombre-Virus, Black-Creek-Canal-Virus, Bayou-Virus, New-York-Virus, Andes-Virus und Laguna-Negra-Virus, ausgewählt ist.

15. Multivalenter Impfstoff nach Anspruch 14, weiterhin umfassend eine Zusammensetzung, umfassend ein Trägerteilchen mit einer oder mehreren DNS-Sequenzen, aufgebracht auf das Trägerteilchen, wobei die DNS-Sequenz einen Promotor, operativ in den Zellen eines Säugers, und eine Protein-Codierungsregion, codierend für eine Determinante eines zweiten Hantavirus, verschieden von dem ersten Hantavirus, umfaßt, wobei das zweite Hantavirus aus der Gruppe ausgewählt ist, bestehend aus Seoul-Virus, Dobrava-Virus, Puumala-Virus, Hantaan-Virus, Sin-Nombre-Virus, Black-Creek-Canal-Virus, Bayou-Virus, New-York-Virus, Andes-Virus und Laguna-Negra-Virus.

## Revendications

1. Composition de matière comprenant une particule porteuse; et une séquence d'ADN enrobée sur la particule porteuse, la séquence d'ADN comprenant un promoteur opératoire dans les cellules d'un mammifère et une région codant une protéine codant pour un déterminant d'une protéine d'hantavirus.

2. La composition de la revendication 1, dans laquelle ladite région codant une protéine code une protéine sélectionnée parmi le groupe consistant en protéines du segment génique M et en protéines du segment génique S.

3. La composition de la revendication 1, dans laquelle ledit hantavirus est sélectionné parmi le groupe consistant en virus Séoul, virus Dobrava, virus Puumala, virus Hantaan, virus Sin Nombre, virus Black Creek Canal, virus Bayou, virus de New York, virus des Andes et virus Laguna Negra.

4. La composition de la revendication 3, dans laquelle l'hantavirus est le virus Séoul.

5. La composition de la revendication 4, dans laquelle la région codant une protéine comprend la SEQ ID NO:1.

6. La composition de la revendication 4, dans laquelle la région codant une protéine comprend la SEQ ID NO:2.

7. La composition de la revendication 1, dans laquelle ladite séquence d'ADN comprend pWRG-SEO-M.

8. La composition de la revendication 1, dans laquelle ladite séquence d'ADN comprend pWRG-SEO-S.

9. Utilisation d'une composition selon l'une quelconque des revendications 1 à 8, dans la préparation d'un vaccin contre une infection à hantavirus.

10. Utilisation d'une composition selon l'une quelconque des revendications 1 à 8, dans la préparation d'un vaccin contre une infection par ledit hantavirus.

11. Utilisation d'une composition selon l'une quelconque des revendications 1, 4, 5 et 6, où ledit hantavirus est un premier hantavirus, dans la préparation d'un vaccin contre une infection par un deuxième hantavirus.

12. Utilisation selon la revendication 11, où le premier hantavirus est le virus Séoul et le deuxième hantavirus est le virus Dobrava ou le virus Hantaan.

13. Vaccin contre une infection à hantavirus comprenant la composition de l'une quelconque des revendications 1 à 8.

14. Vaccin multivalent pour la protection contre une infection à plus d'un hantavirus, comprenant une composition de matière comprenant une particule porteuse ayant une ou plusieurs séquences d'ADN enrobées sur la particule porteuse, la séquence d'ADN comprenant un promoteur opératoire dans les cellules d'un mammifère et une région codant une protéine codant pour un déterminant d'une protéine d'un premier hantavirus, ledit hantavirus étant sélectionné parmi le groupe consistant en SEOV, Dobrava, Puumala, Hantaan, virus Sin Nombre, virus Black Creek Canal, virus Bayou, virus de New York, virus des Andes et virus Laguna Negra.

15. Le vaccin multivalent de la revendication 14, comprenant en outre une composition comprenant une particule porteuse ayant une ou plusieurs séquences d'ADN enrobées sur la particule porteuse, la séquence d'ADN comprenant un promoteur opératoire dans les cellules d'un mammifère et une région codant une protéine codant pour un déterminant d'un deuxième hantavirus différent dudit premier hantavirus, ledit deuxième hantavirus étant sélectionné parmi le groupe consistant en virus Séoul, virus Dobrava, virus Puumala, virus Hantaan, virus Sin Nombre, virus Black Creek Canal, virus Bayou, virus de New York, virus des Andes et virus Laguna Negra.
